(19) Europäisches Patentamt
European Patent Office
Office européen des brevets

(11) Publication number: 0 089 139 A2

# (12) EUROPEAN PATENT APPLICATION

(21) Application number: 83301073.9

(22) Date of filing: 01.03.83

(51) Int. Cl.³: C 07 D 487/04
A 61 K 31/40
//A61K31/43, A61K31/545,
(C07D487/04, 209/00, 205/00)

(30) Priority: 16.03.82 GB 8207618

(43) Date of publication of application: 21.09.83 Bulletin 83/38

(84) Designated Contracting States: CH DE FR GB IT LI NL

(71) Applicant: BEECHAM GROUP PLC
Beecham House Great West Road
Brentford Middlesex(GB)

(72) Inventor: Southgate, Robert
7 Tillets Lane
Warnham West Sussex(GB)

(72) Inventor: Brown, Pamela
17 Greenhill Gardens
Merrow Guilford Surrey(GB)

(74) Representative: Hesketh, Alan, Dr. et al,
European Patent Attorney Beecham Pharmaceuticals
Great Burgh Yew Tree Bottom Road
Epsom, Surrey, KT18 5XQ(GB)

## (54) Antibiotics, their preparation and use.

(57) 1. A compound of the formula (II):

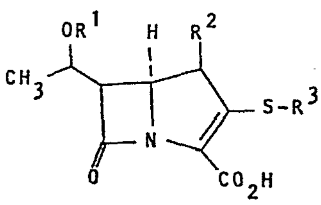

(II)

or a pharmaceutically acceptable salt or pharmaceutically acceptable ester thereof, wherein $R^1$ is hydrogen, $-CHO$ or $-SO_3H$ or a pharmaceutically acceptable salt thereof, $R^2$ is $C_{1-6}$ alkyl or benzyl, and $R^3$ is an optionally substituted pyrimidinyl group. Processes for the preparation of such compounds and pharmaceutical compositions containing these compounds are also disclosed.

EP 0 089 139 A2

Croydon Printing Company Ltd.

# ANTIBIOTICS, THEIR PREPARATION AND USE

This invention relates to novel 4-substituted 7-oxo-1-azabicyclo[3.2.0]heptene derivatives and in particular to such derivatives with a C-3 pyrimidinyl thio substituent. This invention further relates to processes for their preparation and to compositions containing them. These derivatives are of use in the treatment of bacterial infection.

European Patent Application Publication No. 00010317 discloses a wide range of synthetic antibacterial agents of the formula (I):

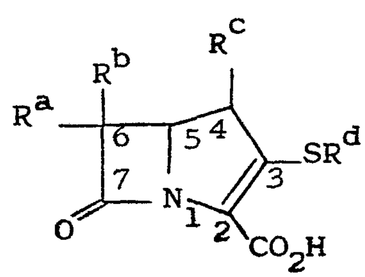

(I)

wherein $R^a$, $R^b$, $R^c$ and $R^d$ independently represent a variety of substituents.

We have now found that compounds within formula (I) having a C-3 pyrimidinylthio substituent show superior efficacy, for example antibacterial activity and/or stability than related compounds specifically

disclosed in the aforementioned European Patent Application.

Accordingly, the present invention provides the compounds of the formula (II):

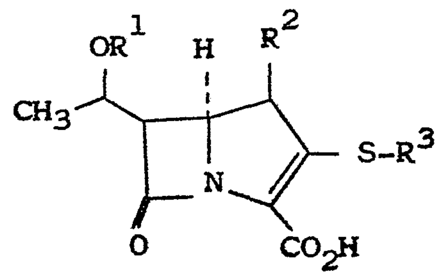

(II)

and pharmaceutically acceptable salts and pharmaceutically acceptable esters thereof, wherein $R^1$ is hydrogen, -CHO or $-SO_3H$ or a pharmaceutically acceptable salt thereof, $R^2$ is $C_{1-6}$ alkyl or benzyl, and $R^3$ is an optionally substituted pyrimidinyl group.

Suitably $R^1$ is $-SO_3H$ or a pharmaceutically acceptable salt thereof such as an alkali metal salt, for example the sodium or potassium salt.

Preferably $R^1$ is a hydrogen atom.

In a further aspect $R^1$ is -CHO, which compounds are active in their own right but it is possible that they are converted <u>in vivo</u> to compounds wherein $R^1$ is hydrogen.

Suitably $R^2$ is $C_{1-6}$ alkyl such as methyl, ethyl, n-propyl, isopropyl, n-butyl, sec-butyl and isobutyl. Suitably also $R^2$ is benzyl.

Preferably $R^2$ is methyl.

Suitably $R^3$ may represent pyrimidin-4-yl or pyrimidin-2-yl, of these pyrimidin-2-yl is preferred.

Suitable substituents for the pyrimidinyl ring include $C_{1-6}$ alkyl such as methyl and ethyl, $C_{1-6}$ alkoxy such as methoxy, nitro, halo such as chloro, amino and $C_{1-6}$ alkanoylamino such as acetamido.

In a preferred aspect the pyrimidinyl ring is unsubstituted.

Thus, a favoured sub-group of compounds is that of the formula (III):

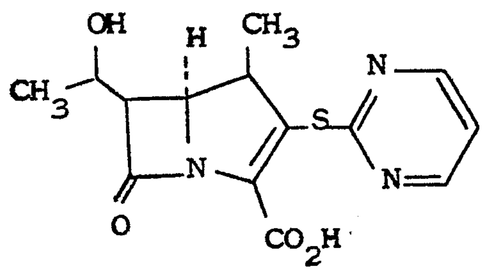

(III)

or pharmaceutically acceptable salts thereof.

The compounds of the formulae (II) - (III) may have R or S stereochemistry at the C-8 position (that is the α-carbon atom of the C-6 substituent) or may be in the form of mixtures thereof.

The compound of the formulae (II) - (III) may be provided with a cis-configuration of the C-5 and C-6 protons, or they may be provided with a trans-configuration of the C-5 and C-6 protons. Alternatively, they are provided as a mixture of cis and trans forms.

The group $R^2$ in the compounds of the formula (II) may have the α- or β- configuration at the C-4 position or may be provided as a mixture thereof. In a favoured aspect $R^2$ in the compounds of the formula (II) has an α-configuration.

Suitable pharmaceutically acceptable salts include those of the alkali and alkaline earth metals, of these the sodium and potassium salts are preferred. These pharmaceutically acceptable salts may be formed at the C-2 carboxy and/or at the C-6 sulphonato-oxyethyl moiety (if present). Thus compounds of the formula (II) wherein $R^1$ is $-SO_3H$ or a pharmaceutically acceptable salt thereof, may be in the form of a di-salt such as the di- sodium or di- potassium salt, or may be in the form of a mono- salt of an in-vivo hydrolysable ester, or may be in the form of a mono-salt of an acid or may be in the form of a di- acid.

Non-pharmaceutically acceptable salts of the compounds of the formula (II) are also of use as they may be converted to the compounds of the formula (II) or a pharmaceutically acceptable salt or ester thereof.

Suitable esters of the compounds of the formula (II) include those cleavable by biological methods such as in-vivo hydrolysis, and those cleavable by chemical methods such as hydrogenolysis, hydrolysis, electrolysis or photolysis.

Suitable esterifying groups include those of the sub-formulae (a) - (f):

$-CHA^1A^2$ (a)

$-CHA^3A^4$ (b)

$-CHA^5-OCOA^6$ (c)

$-CHA^5-OA^7$ (d)

$-SiA^8A^9A^{10}$ (e)

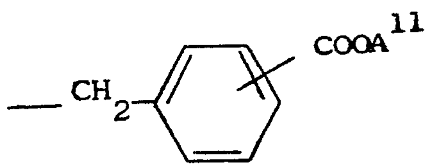
(f)

wherein $A^1$ is a hydrogen atom, $C_{1-6}$ alkanoyl or an alkyl, alkenyl or alkynyl group of up to 3 carbon atoms; $A^2$ is a hydrogen atom or a methyl group; $A^3$ is a phenyl group or a phenyl group substituted by a fluorine, chlorine or bromine atom or a nitro, methyl or methoxy group; $A^4$ is a hydrogen atom or a phenyl group or a phenyl group substituted by a fluorine, chlorine or bromine atom or a nitro, methyl or methoxy group; $A^5$ is a hydrogen atom or a methyl group; $A^6$ is a $C_{1-4}$ alkyl, phenyl or $C_{1-4}$ alkoxy group or $A^5$ is joined to $A^6$ to form a phthalidyl, dimethylphthalidyl or dimethoxyphthalidyl group; $A^7$ is a $C_{1-4}$ alkyl, phenyl, chlorophenyl or nitrophenyl group; $A^8$ is a $C_{1-4}$ alkyl or phenyl group; $A^9$ is a $C_{1-4}$ alkyl or phenyl group; $A^{10}$ is $C_{1-4}$ alkyl; and $A^{11}$ is $C_{1-4}$ alkyl: or $CHA^1A^2$ is a phenacylmethyl or bromophenacylmethyl group.

Favourably $A^1$ is a hydrogen atom or a methyl, ethyl, vinyl or ethenyl group. Favourably $A^2$ is a hydrogen atom. Favourably $A^3$ is a phenyl, p-bromophenyl, p-methoxyphenyl or p-nitrophenyl group.

Favourably $A^4$ is a hydrogen atom. Favourably $A^6$ is a methyl, t-butyl or ethoxy group or is joined to $A^5$. Favourably $A^7$ is a methyl group.

Preferred groups of the sub-formula (a) include the methyl, ethyl and acetonyl groups.

Preferred groups of the sub-formula (b) include the benzyl and p-nitrobenzyl groups.

Preferred groups of the sub-formula (c) include the acetoxymethyl, pivaloyloxymethyl, α-ethoxycarbonyloxymethyl and phthalidyl groups.

A preferred group of the sub-formula (d) is the methoxymethyl group.

Preferred groups of the sub-formula (e) include the trimethylsilyl, tert-butyldimethylsilyl and tert-butyldiphenylsilyl groups.

A preferred group of the sub-formula (f) is p-methoxycarbonylbenzyl.

Particularly preferred esterifying groups are the p-nitrobenzyl and phthalidyl groups.

Pharmaceutically acceptable in-vivo hydrolysable esters are those esters which hydrolyse in the human body to produce the parent acid or its salt. Such esters may be identified by administration to a test animal such as a rat or mouse by intravenous administration and thereafter examining the test animal's body fluids for the presence of the compound of the formula (II) or its salt.

Suitable esters of this type include those of sub-formula (c) as hereinbefore defined.

The compounds of this invention may be employed in the treatment of bacterial infection. Thus the present invention also provides a pharmaceutical composition which comprises a compound of the formula (II) or a pharmaceutically acceptable salt or ester thereof and a pharmaceutically acceptable carrier. Preferably any such ester is in-vivo hydrolysable.

The compositions of this invention may be prepared by conventional methods of preparing antibiotic compositions and in conventional manner may be adapted for oral, topical or parenteral administration.

Aptly, the compositions of this invention are in the form of a unit-dose composition adapted for oral administration.

Alternatively the compositions of this invention are in the form of a unit-dose composition adapted for administration by injection.

Unit dose forms according to this invention will normally contain from 50 to 1000 mg of a compound of this invention, for example about 62.5, 100, 150, 200, 250, 500 or 750 mg. Such compositions may be administered from 1 to 6 times a day or more conveniently 2, 3 or 4 times a day so that the total daily dose for a 70 kg adult is about 200 to 2000 mg, for example about 400, 600, 750, 1000 or 1500 mg.

The compositions of this invention may be used to treat bacterial infection in animals such as mammals

including humans, for example infections of the respiratory tract, urinary tract or soft tissues, or mastitis in cattle.

The carriers used in the compositions of this invention may include diluents, binders, disintegrants, lubricants, colours, flavouring agents or preservatives in conventional manner. Thus suitable agents include lactose, starch, sucrose, calcium phosphate, sorbitol, polyvinylpyrrolidone, acacia, gelatin, tragacanth, potato starch or polyvinylpolypyrrolidone, magnesium stearate or sodium lauryl sulphate.

Orally administrable forms of the compositions of this invention are most suitably in the form of unit-dose units such as tablets or capsules.

The present invention also provides synergistic pharmaceutical compositions which comprise a pharmaceutical composition as hereinbefore described and a penicillin or cephalosporin.

Suitable penicillins for inclusion in the synergistic compositions of this invention include benzylpenicillin, phenoxymethylpenicillin, ampicillin, amoxycillin, ticarcillin, suncillin, sulbenicillin, azlocillin or mezlocillin; in particular amoxycillin as its sodium salt or trihydrate is preferred.

Suitable cephalosporins for inclusion in the synergistic compositions of this invention include cephaloridine, cefazolin and cephradine.

The penicillin or cephalosporin is generally utilised in its conventionally administered amount.

The weight ratio between the compound of this invention and penicillin or cephalosporin is generally from 10:1 to 1:10, more usually from 5:1 to 1:5 and normally from 3:1 to 1:3.

In an alternative aspect the present invention provides a process for the preparation of a compound of the formula (II) or pharmaceutically acceptable salt or pharmaceutically acceptable ester thereof, which process comprises the elimination of the elements of $R^4R^5R^6P{=}O$ from an ester of a compound of the formula (IV):

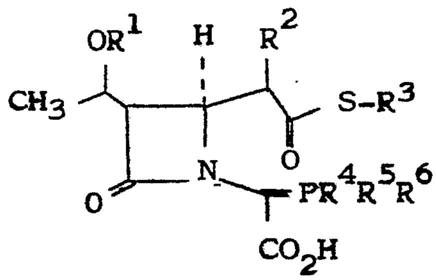

(IV)

wherein $R^1$ is as hereinbefore defined or protected derivative thereof, $R^2$ and $R^3$ are as hereinbefore defined, and $R^4$, $R^5$ and $R^6$ are independently phenyl or methoxyphenyl, and thereafter if necessary:

i) converting a compound of the formula (II) wherein $R^1$ is -CHO to a compound of the formula (II) wherein $R^1$ is hydrogen,

ii) converting the ester to an acid, pharmaceutically acceptable salt or pharmaceutically acceptable ester,

iii) removing any protecting group.

In all of the processes disclosed in this specification, $R^1$ may be optionally protected. Suitable protected derivatives of the group $R^1$ are those conventional in the art, for example, groups cleavable by hydrolysis such as formate or groups cleavable by hydrogenation such as benzyloxycarbonyloxy and p-nitrobenzyloxycarbonyloxy.

Suitably this process is performed by heating the ester of the compound of the formula (IV) in an inert solvent, for example at temperatures of 90°C to 120°C and more suitably 100°C to 110°C in a solvent such as toluene, preferably under substantially anhydrous conditions.

Suitable ester-forming groups are those which may be removed under conventional conditions. Such groups include benzyl, p-methoxybenzyl, 2,4,6-trimethylbenzyl, 3,5-di-t-butyl-4-hydroxybenzyl, benzoylmethyl, p-nitrobenzyl, 4-pyridylmethyl, 2,2,2-trichloroethyl, 2,2,2-tribromoethyl, acetonyl, t-butyl, t-amyl, diphenylmethyl, triphenylmethyl, adamantyl, 2-benzyloxyphenyl, 4-methylthiophenyl, tetrahydrofur-2-yl, tetrahydropyran-2-yl, pentachlorophenyl, p-toluenesulphonylethyl, methoxymethyl, a silyl, stannyl or phosphorus-containing group, an oxime radical of formula -N=CHR° where R° is aryl or heterocyclic, or an in-vivo hydrolysable ester.

The carboxyl group may be regenerated from any of the above esters by usual methods appropriate to the particular group, for example, acid- and base-catalysed hydrolysis, or by enzymically - catalysed hydrolysis, or by hydrogenation. The hydrolysis must of course be carried out under conditions to which the

0089139

groups on the rest of the molecule are stable. For the preparation of a compound of formula (II) in the form of a pharmaceutically acceptable ester, it is convenient to employ a compound of formula (IV) wherein the desired ester group is present.

The preparation of the compounds of the formula (IV) may be effected by the reaction of a compound of the formula (V):

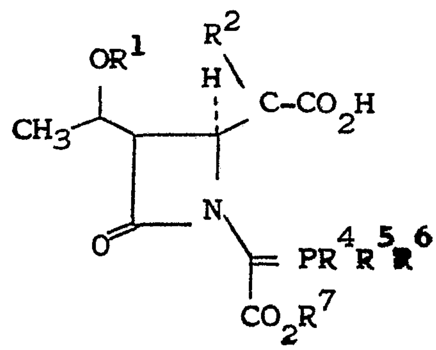

(V)

wherein $R^1$, $R^2$, $R^4$, $R^5$, and $R^6$ are as defined in relation to formula (IV) and $R^7$ is an esterifying group; with a di($C_{1-6}$)alkylphosphorochloridate or thionyl chloride and a tri($C_{1-6}$)alkylamine followed by reaction with a derivative of the formula (VI):

$$L^{\oplus} \ominus SR^3 \qquad (VI)$$

wherein $L^+$ is a lithium, sodium, silver or thallium (I) cation or an ammonium ion substituted by up to three $C_{1-6}$ alkyl groups, and $R^3$ is as hereinbefore defined.

When $L^+$ is a substituted ammonium ion, it is preferably a tertiary ammonium ion, such as the triethylammonium ion. It is conveniently generated in situ by the reaction of a compound of the formula $HSR^3$ with an amine, preferably a tertiary amine.

Preferably $L^+$ is a lithium cation, a thallium (I) cation or a silver cation.

A particularly suitable di-loweralkylphosphoro-chloride is diethylphosphorochloridate. A particularly suitable tri-loweralkylamine is triethylamine.

The reaction is generally carried out in an inert organic solvent such as tetrahydrofuran at a non-extreme temperature such as 0°C to 40°C, for example 15° to 25°C.

The compound of the formula (V) may be prepared by the reaction of the compound of the formula (VII):

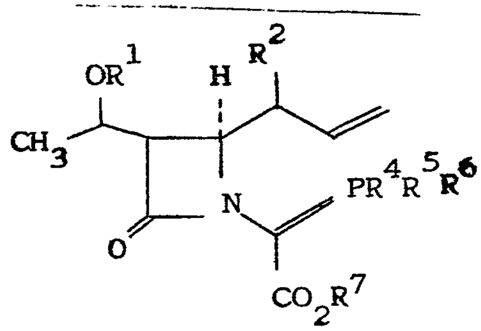

(VII)

wherein $R^1$, $R^2$, $R^4$, $R^5$, $R^6$ and $R^7$ are as hereinbefore defined, with ozone in the presence of trifluoroacetic acid followed by m-chloroperbenzoic acid.

The ozonolysis is generally performed at a depressed temperature such as -40°C to -80°C, for example about -70°C and in solution in an inert solvent such as methylene chloride. Excess ozone is removed by flushing with an inert gas and thereafter a solution of the peracid is added to the reaction mixture.

0089139

The compound of the formula (VII) may be prepared by the acylation or alkylation of a compound of the formula (VIII):

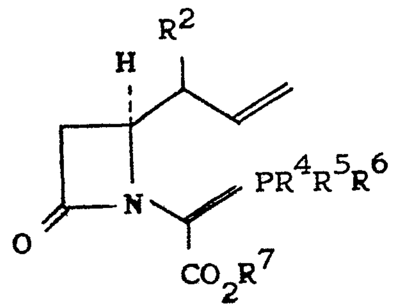

(VIII)

wherein $R^2$, $R^4$, $R^5$, $R^6$ and $R^7$ are as hereinbefore defined. Suitably the compound of the formula (VIII) is treated with a strong base of low nucleophilicity such as butyl lithium, lithium di-iso-propylamide or lithium iso-propyl-cyclohexylamide, in a solvent such as tetrahydrofuran, hexane, dimethoxyethane, dimethylformamide or hexamethylphosphorus triamide, normally at a temperature below 0°C, preferably -80°C to -60°C, using the desired electrophilic agent R - X wherein X is a leaving group such as mesylate, tosylate, chloro, bromo or iodo. Alternatively, the electrophilic agent is an aldehyde or ketone. A favoured acylating agent is acetylimidazole.

The compound of the formula (VIII) may be prepared by the reaction of $PR^4 R^5 R^6$ wherein $R^4 R^5 R^6$ are as hereinbefore defined and the compound of the formula (IX):

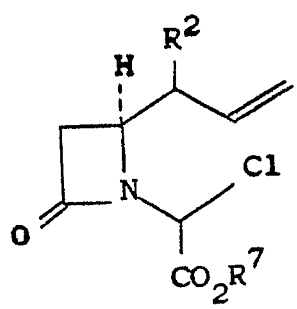

(IX)

wherein $R^2$ and $R^7$ are as hereinbefore defined.

This reaction is normally effected in the presence of at least one equivalent of a base of relatively low nucleophilicity such as 2,6-lutidine at an ambient temperature in a dry solvent such as dioxan, tetrahydrofuran or the like.

The ester of the compound of the formula (IX) may be prepared from the corresponding ester of the carbinol of the formula (X):

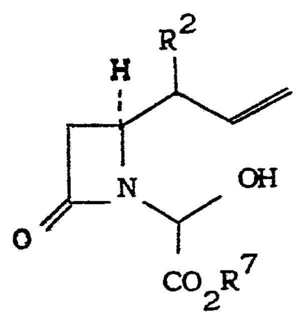

(X)

wherein $R^2$ and $R^7$ are as hereinbefore described, by reaction with thionyl chloride.

This reaction is also normally effected in the presence of at least one equivalent of a base of relatively low nucleophilicity in a dry solvent such as dioxan or tetrahydrofuran but in this instance the reaction is performed at a depressed temperature, for example -30° to -10°C.

The ester of the preceding carbinol may be prepared by the reaction of the compound of the formula (XI):

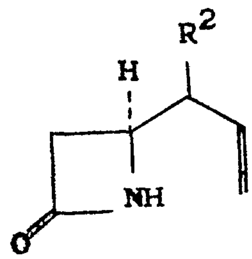

(XI)

wherein $R^2$ is as hereinbefore defined, with a glyoxylic acid ester.

Normally this reaction is carried out in an inert solvent at an elevated temperature, for example, in dry benzene under reflux.

The esters of the compounds of the formula (X) may also be prepared by esterification of a salt of the compound of the formula (X) in conventional manner.

Suitable methods include the reaction of alkali metal salt such as a sodium or potassium salt with a reactive halide or sulphonate ester such as a bromide, chloride, mesylate, tosylate or the like. Such esterifications may be carried out under conventional conditions, for example in dimethylformamide at room temperature.

The salt of compound of the formula (X) may be prepared by neutralisation of the compound of the formula (X) for example, with an alkali metal carbonate or bicarbonate, for example, sodium or potassium carbonate.

The compound of formula (X) may be prepared by the reaction of glyoxylic acid with the compounds of the formula (XI) as hereinbefore defined.

In an alternative aspect the compound of the formula (VII) may be prepared by the reaction of a compound of the formulae (XII) or (XIII):

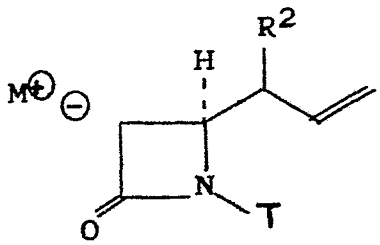

(XII)

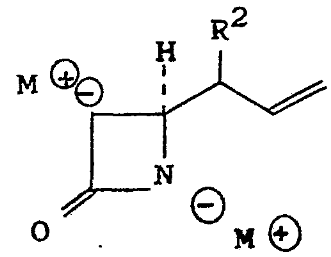

(XIII)

wherein $R^2$ is as hereinbefore defined, T is a protecting group for example, a silyl protecting group such as dimethyl-t-butylsilyl and $M^+$ is a counter-ion for example, lithium; with an acylating agent; and thereafter removing any protecting group T and converting the -NH moiety to $-N-C(CO_2R^7)=PR^4R^5R^6$ in analogous manner to that described hereinbefore.

Generally the compound of the formula (XIII) is generated and utilised in situ. Then the compound of the formula (XI) may be treated with two equivalents of n-butyl lithium in tetrahydrofuran at a low temperature. The dianion may be quenched by the addition of an acylating agent.

Generally the compound of the formula (XII) is generated and used in situ. Thus, the compound of the formula (XI) may be treated with two equivalents of n-butyl lithium in tetrahydrofuran at a low temperature. The anion may be quenched by the addition of an acylating agent. The protecting group T may be introduced and removed in conventional manner, for

example, 4-(1-methyl)allyl-1-t-butyldimethylsilyl-azetidin-2-one may be prepared by the reaction of 4-(1-methyl)allyl- azetidin-2-one with t-butyldimethylsilyl chloride and triethylamine in an inert solvent. The t-butyldimethylsilyl protecting group may be removed when necessary on treatment with potassium fluoride and a crown ether in an inert solvent.

The compound of the formula (V) may also be prepared by the ozonolysis of an ester of a compound of the formula (XIV):

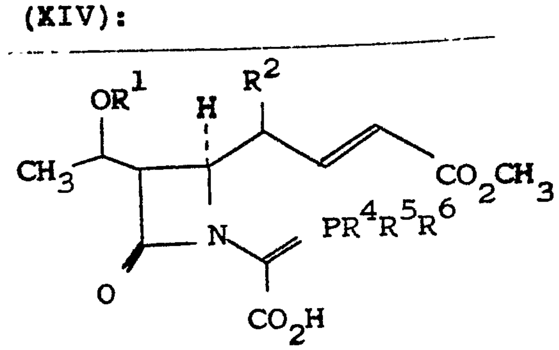

(XIV)

wherein $R^1$, $R^2$, $R^4$, $R^5$ and $R^6$ are as hereinbefore defined. The ozonolysis is generally performed in the presence of trifluoroacetic acid in dichloromethane or ethyl acetate at -70°C, followed by m-chloroperbenzoic acid.

The ester of the compound of the formula (XIV) may be prepared from a compound of the formula (XV ) in an analogous manner to that described hereinabove:

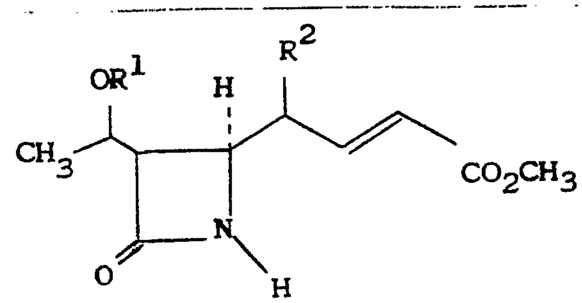

(XV)

wherein $R^1$ and $R^2$ are as hereinbefore defined.

The compound of the formula (XV) may be prepared by the oxidation of a compound (XVI):

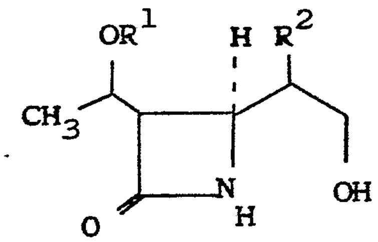

(XVI)

wherein $R^1$ and $R^2$ are as hereinbefore defined, with pyridinium chlorochromate and thereafter reacting the thus produced aldehyde with $Ph_3P{=}CHCO_2CH_3$. The oxidation may be performed at ambient temperature in dichloromethane. When the oxidation is judged to be complete (for example by t.l.c) the reaction mixture may be filtered and the phosphorane added to the filtrate.

The compound of the formula (XVI) may be prepared by mild acid hydrolysis of a compound of the formula (XVII):

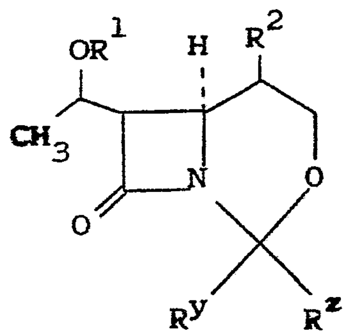

(XVII)

wherein $R^1$ and $R^2$ are as hereinbefore defined and $R^y$ and $R^z$ are independently $C_{1-6}$ alkyl such as methyl, or are joined together to form a substituted or unsubstituted spirocycloalkyl ring, for example, spiro-cyclohexyl.

In one aspect compounds of the formula (XVII) may be prepared by methods analogous to those described in UK Patent Application No. 2013667 ie according to the Scheme:

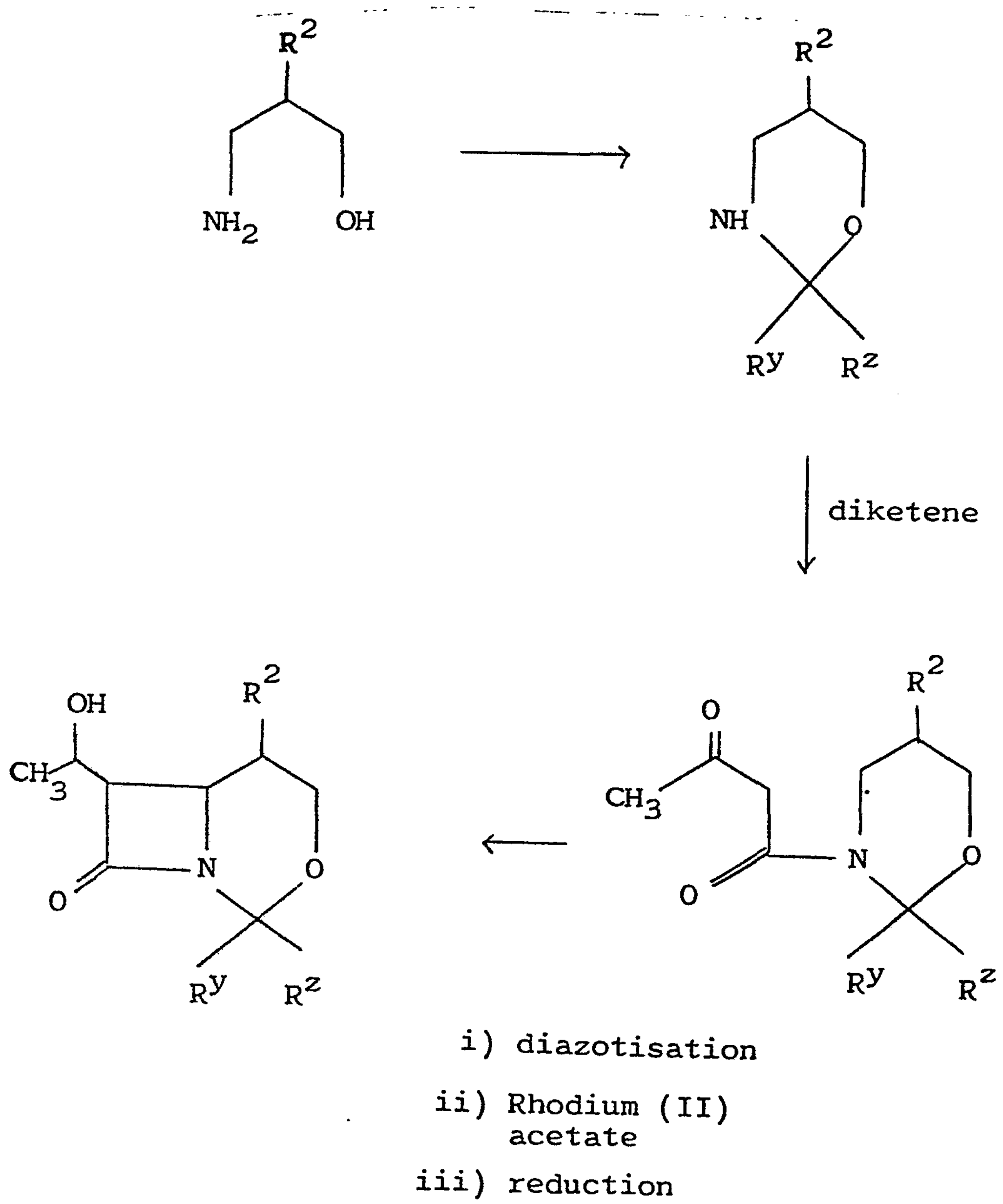

A preferred aspect of this process is that surprisingly the ring closing cyclisation with Rhodium acetate is stereocontrolled giving 90% of one pair of diastereoisomers, which may be readily separated from the remaining diastereoisomers. In addition, if the starting material, the alcohol $NH_2CH_2CHR^2OH$ is optically active then operation of this Scheme results in an optically active bicyclic product which may be elaborated through the hereinbefore described processes to provide an optically active compound of the formula (II).

The following Examples illustrate the invention. In Examples 4-16 inclusive one enantiomer only is depicted for convenience.

Example 1

3-bromo-2-methyl propan-1-ol

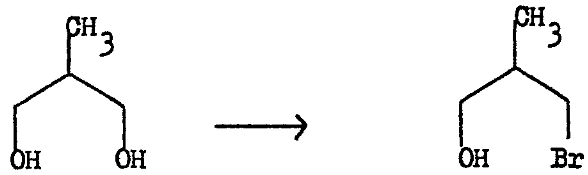

(1) (2)

3-Bromo-2-methyl propan-1-ol was prepared according to the method of Searles et al., J. Org. Chem., 1959, 24, 1839. 2-methyl propan-1,3-diol (1), (1.78 g) (E.R. Nelson, M. Maienthal et al., J. Amer. Chem. Soc., 1957, 79, 3467) was treated with acetic acid (4 ml) and 48% hydrobromic acid (0.1 ml) and heated to reflux for 20 minutes. A solution of 45% hydrogen bromide in acetic acid (4 ml) and acetic acid (4 ml) was added to the refluxing reaction over a period of 3 h., and refluxing continued for a further 8 h. The product was concentrated under reduced pressure and treated with 48% hydrobromic acid (0.1 ml) in ethanol (7 ml). The solvents were distilled under atmospheric pressure, and the procedure repeated twice until the distillate no longer contained ethyl acetate. The residue was distilled under reduced pressure to yield 3-bromo-2-methyl propan-1-ol (2) (1.67 g); b.p. 88 - 90°C (20 mm); $\nu_{max.}$ ($CH_2Cl_2$) 3620, 3300, 2960, 1460, 1380, 1230, 1030, 980, 650, 620 $cm^{-1}$; $\delta$ ($CDCl_3$) 1.00 (3H, d, J = 7Hz, $CH_3$); 1.80 - 2.30 (1H, m, C(2)H); 3.50 (2H, d, J = 6Hz); 3.62 (2H, d, J = 6Hz); 5.95 (1H, br.s. $D_2O$ exch ., OH).

Example 2

3-azido-2-methyl propan-1-ol

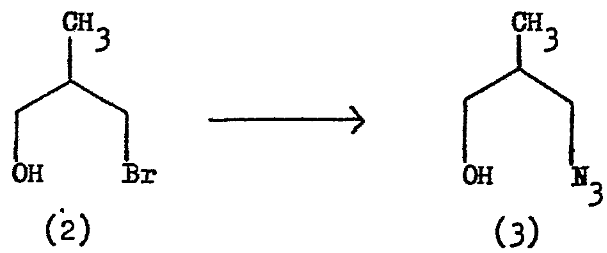

(2) (3)

A solution of 3-bromo-2-methyl propan-1-ol (2) (12.5 g) in dry dimethyl formamide (50 ml) was treated with finely-ground sodium azide (10 g) and tetramethylguanidinium azide (2 g). The reaction mixture was stirred at 90°C for 24 hours, adding a further portion of sodium azide (5 g) after 16 h. The reaction mixture was poured into water (75 ml), the product extracted into ethyl acetate and the organic phase washed with 3% sodium bicarbonate solution (5 x 25 ml). The combined aqueous extracts were back-extracted into ethyl acetate (50 ml) and the combined organic extracts dried over anhydrous magnesium sulphate. Evaporation of the solvent gave 3-azido-2-methyl propan-1-ol (3) (9.1 g) as a colourless oil which was not purified further; $\nu_{max.}$ ($CHCl_3$) 3620, 3420, 2120, 1460, 1390, 1280, 1030 $cm^{-1}$; $\delta$ (ppm, $CDCl_3$) 1.00 (3H, d, J = 7Hz, $CH_3$); 1.70 - 2.20 (1H, m, C(2)H); 3.10 (1H, br.s., OH), 3.40 (2H, d, J = 6Hz), 3.55 (2H, d, J = 6Hz).

Example 3

3-amino-2-methyl propan-1-ol

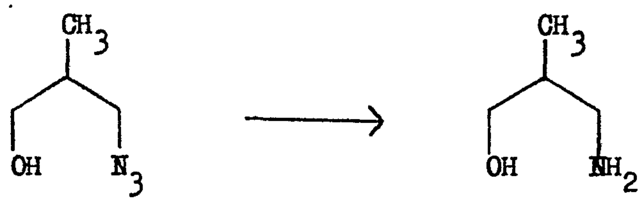

(3) (4)

3-azido-2-methyl propan-1-ol (3) (9.85 g) was dissolved in ethanol (120 ml), treated with 10% paladium on charcoal (0.5 g) and hydrogenated at atmospheric pressure in an apparatus containing 750 ml hydrogen. The reaction was carried out for 4 hours, replacing the gas in the vessel with fresh hydrogen at hourly intervals. The reaction mixture was filtered through Kieselguhr and the solvent evaporated under reduced pressure to give 3-amino-2-methyl propan-1-ol (4) as a colourless oil which was not purified further; $\nu_{max.}$ ($CH_2Cl_2$) 3620, 3380, 3300 br., 1585, 1460, 1390, 1030 $cm^{-1}$; δ (ppm., $CDCl_3$) 0.90 (3H, d, J = 7Hz, $CH_3$); 1.46 (1H, m, C(2) H); 2.72 (2H, d, J = 6Hz, $CH_2NH_2$); 2.97 (2H, s, $NH_2$); 3.25 (1H, br. s. OH), 3.50 (2H, d, J = 6Hz, $CH_2OH$).

Example 4

1-[3-Methyl-1-oxa-5-azaspiro[5.5]undecan-5-yl]butan-1,3-dione

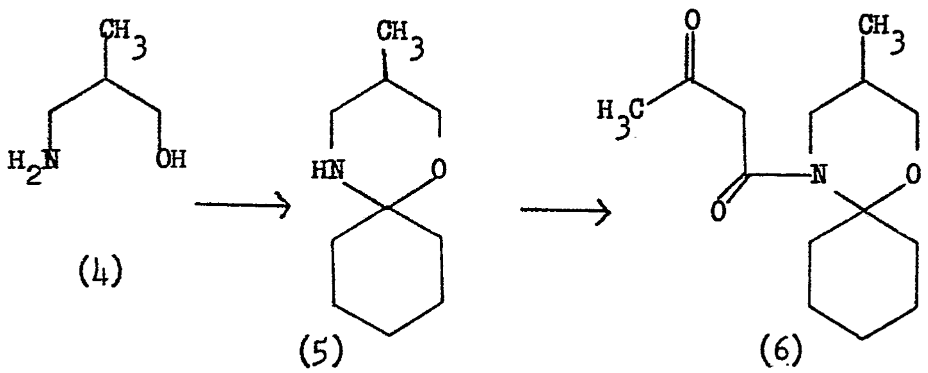

A solution of 3-amino-2-methyl propan-1-ol (4) (0.400 g) in dry benzene (10 ml) was treated with cyclohexanone (0.450 g) and heated at reflux in an apparatus with provision for water removal for 5 hours. The product was concentrated to give the crude 3-methyl-1-oxa-5-azospiro[5.5]undecane (5) as a yellow oil; $\nu_{max.}$ ($CH_2Cl_2$) 3450, 1660 (weak, Schiffs base); 1460, 1450, 1160, 1040 $cm^{-1}$. This material was redissolved in dry benzene (10 ml) and stirred and cooled in an ice-bath, treated with diketene (0.37 ml) and stirring continued at $0^oC$ for 1 h followed by room temperature for 1 h. The solvent was evaporated and the residue chromatographed on Kieselgel 60 (<230 mesh ASTM) eluting with hexane grading to 1:1 ethyl acetate/hexane, to give 1-[3-methyl-1-oxa-5-azaspiro[5.5]undecan-5-yl butan-1,3-dione (6) (0.400 g) as a yellow oil; $\nu_{max}$ ($CH_2Cl_2$) 1725, 1640, 1450, 1410, 1550 $cm^{-1}$; δ (ppm, $CDCl_3$) 0.96 (3H, d, J = 7Hz, $\underline{CH_3}CH$); 1.25 - 2.70 (10 H, m, cyclohexyl); 2.10 - 2.20 (1H, m, $CH_3\underline{CH}$); 2.27 (3H, s, $C(O)CH_3$); 3.10 (1H, dd, J = 12, 7Hz, $\underline{CHa}HbN$); 3.27 (1H, dd, J = 11, 7 Hz, $\underline{CHa}HbO$); 3.34 (1H, dd, J = 12, 7Hz, $CHa\underline{Hb}$ N); 3.50 (2H, d, J = 2.5 Hz, $COCH_2CO$); 3.94 (1H, dd, J = 11, 7 Hz, $CHa\underline{Hb}O$).

Example 5

1-[3-methyl-1-oxa-5-azaspiro[5.5]undecan-5-yl]-2-diazabutan-1,3-dione

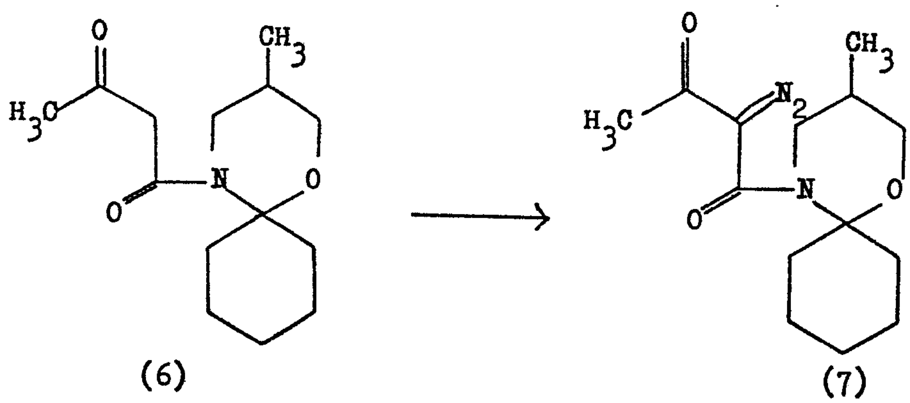

(6) (7)

1-[3-Methyl-1-oxa-5-azaspiro[5.5]undecan-5-yl]butan-1,3-dione (6) (9.7 g) was dissolved in dry toluene (125 ml) and cooled to 0°C. The solution was treated with triethylamine (6 g), piperidine (0.1 g) and mesyl azide (7 g) in toluene (30 ml), stirred at 0°C for 1 hr then allowed to warm to room temperature and stirred for 40 hr. The solvent was evaporated and the residue chromatographed on Kieselgel 60 (<230 mesh ASTM) using 1:4 ethyl acetate/hexane as eluant, to give 1-[3-methyl-1-oxa-5-azaspiro[5.5]undecan-5-yl]-2-diazabutan-1,3-dione (7) (8.76 g) as a yellow oil, contaminated with mesyl azide in approximately 1:1 molar ratio $\nu_{max}$ ($CH_2Cl_2$) 2140 (mesyl azide), 2110 1660 br., 1390, 1365, 1200, 1170, 1060, 965 $cm^{-1}$; δ (ppm, $CDCl_3$); 0.95 (3H, d, J = 7 Hz, $C\underline{H}_3CH$); 1.25 - 2.70 (10 H, m, cyclohexyl - H); 2.15 - 2.29 (1H, m, $CH_3C\underline{H}$); 2.34 (3H, s, $C(O)CH_3$); 3.15 (1H, dd, J = 12, 7 Hz, C$\underline{H}$aHbN); 3.28 - 3.38 (2H, m, CHa$\underline{H}$bN and C$\underline{H}$aHbO); 3.93 (1H, dd, J = 12, 7 Hz, CHa$\underline{H}$bO).

Example 6

(5'RS,6'SR, 7'RS)-7'-Acetyl-5'-methylspiro[cyclohexane-1,2'-[3]oxa[1]azabicyclo[4.2.0]octane]-8'-one.

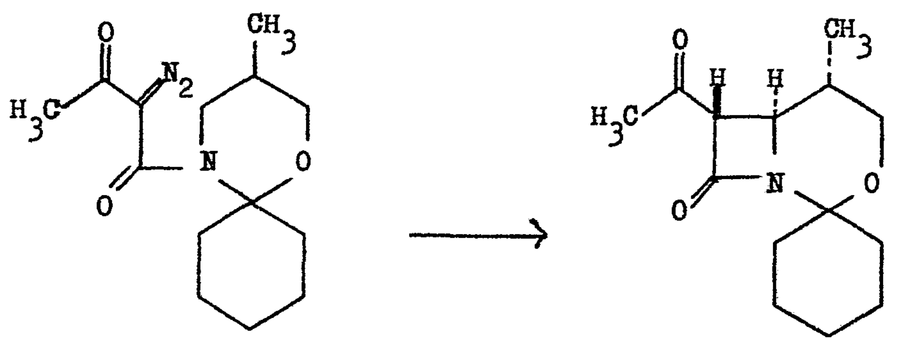

(7) (8)

1-[3-Methyl-1-oxa-5-azaspiro[5.5]undecan-5-yl]-2-diazobutan-1,3-dione (7) (6.13 g) was dissolved in toluene (70 ml) and added to a stirred suspension of rhodium II acetate (0.70 g) in toluene (30 ml) The reaction mixture was stirred at room temperature for 22 h followed by 2 h at 50°C. The mixture was filtered through Kieselguhr, the solvent evaporated and the residue chromatographed on Kieselgel 60 (<230 mesh ASTM) using 1:3 ethyl acetate/hexane as eluant. From the first fractions was obtained the title compound (5' RS, 6'SR, 7'RS)-7'-acetyl-5'-methylspiro[cyclohexane-1,2'-[3]oxa[1]azabicyclo[4.2.0]octane]-8'one (8) (2.902 g) as a pale yellow oil which was crystallised from hexane; m.p. 109 - 110°C; $\nu_{max.}$ ($CH_2Cl_2$) 1775, 1715, 1365, 1200 $cm^{-1}$; δ (ppm, $CDCl_3$) 0.89 (3H, d, J = 7Hz, 5'-$CH_3$); 1.35 - 1.88 (10 H, m, cyclohexyl-H and 5'-H); 2.20 - 2.28 (1H, m, cyclohexyl-H); 2.32 (3H, s, C(O)$C\underline{H}_3$); 3.51 - 3.74 (3H, m, contains [3.59(1H, dd, J = 2, 8Hz, 6'-H); 3.76 (d, J = 12Hz) and 3.72 (d, J = 12Hz part of 2 x ABq of 4'-H]; 3.81 (1H, d, J = 2Hz, 7'-H). Evaporation of the middle fractions gave an oil (0.441 g) containing predominantly (8) with a small amount of the 5'-epimer; (5'RS, 6'RS, 7'SR)-7'-acetyl-5-methylspiro[cyclohexane-1,2'-[3]oxa[1]azabicyclo[4.2.0]octane]-8'-one, from which (8) was obtained pure by crystallisation from hexane.

The later fractions gave an oil (0.460 g) consisting of a 1:1 mixture of (8) and its 5'-epimer. The NMR spectrum of the latter showed, by difference; δ(ppm, $CDCl_3$); 1.14(3H, d, J = 7Hz, 5'-$CH_3$ 1.35-1.92 (10H, m, cyclohexyl and 5'-H); 1.92 - 2.00 (1H, m, cyclohexyl); 2.32 (3H, s, $C(O)CH_3$); 3.56 and 3.62 (1H, 2d, J = 2Hz, 4'-H); 3.97 and 4.02 (1H, 2d, J = 2Hz, 4'-H); 4.02 (1H, d, J = 2Hz, 7'-H); 4.17 (1H, dd, J = 2,5Hz, 6'-H).

Example 7

(5'RS,6'SR,7'RS)-5'-Methyl-7'-[(1SR)-1-hydroxyethyl]spiro[cyclohexane-1,2'-[3]oxa[1]azabicyclo[4.2.0]octane]-8'-one

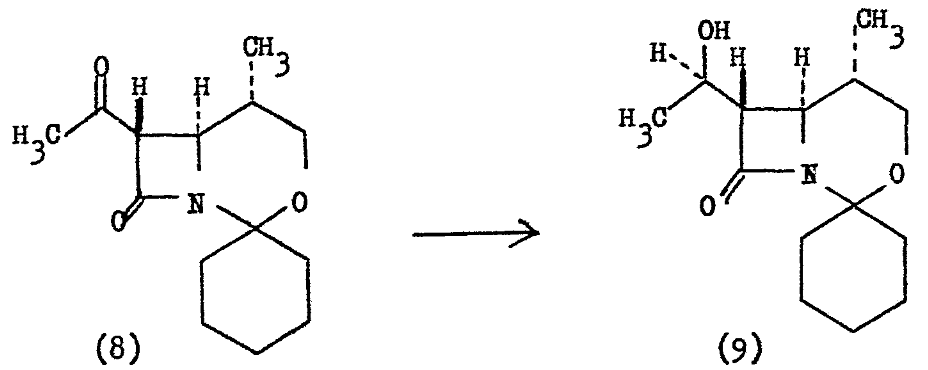

(8) (9)

A solution of (5'RS,6'SR,7'RS)-7'-acetyl-5'-methylspiro[cyclohexane-1,2'-[3]oxa[1]azabicyclo[4.2.0]octane]-8'-one (2.32g) (8) in dry tetrahydrofuran (150 ml) was cooled to 5°C and treated with a 0.5 M solution in tetrahydrofuran of potassium tri-sec-butyl borohydride (20 ml) under an argon atmosphere. The reaction mixture was stirred at 5°C for 3.5 h, and washed with saturated ammonium chloride (2 x 150 ml). The combined aqueous extracts were back-extracted into ethyl acetate and the combined organic phases dried over magnesium sulphate, evaporated and the residue chromatographed on Kieselgel 60 (<230 mesh ASTM) using 1:1 ethyl acetate/hexane as eluant. The product, (5'RS,6'SR, 7'RS)-5'-methyl-7'-[(1SR)-1-hydroxyethyl]spiro[cyclohexane-1,2'-[3]oxa[1]azabicyclo[4.2.0] octane]-8'-one (9) was obtained as a white solid (1.43 g) which could be crystallised from ethyl acetate/hexane; m.p. 133 - 134°C; $\nu_{max.}$ ($CH_2Cl_2$) 3600, 3450, 1745 $cm^{-1}$; δ (ppm, $CDCl_3$) 0.92 (3H, d, J = 6Hz, 5'-$C\underline{H}_3$); 1.32 (3H, d, J = 6Hz, $C(O)CH_3$); 1.35 - 1.88 (11H, m, cyclohexyl, O-H and 5'-H); 2.27 - 2.38 (1H, m, cyclohexyl), 2.79 (1H, dd, J = 5, 1.5Hz, 7'-H); 3.14 (1H, dd, J = 1.5, 10Hz, 6'-H); 3.47 and 3.51 (1H, ABq, J = 12Hz, 4'-H); 3.68 and 3.71 (1H, ABq, J = 12Hz, 4'-H); 4.11 - 4.22 (1H, dq, appears as 6 lines, J = 5Hz, C$\underline{H}$OH). (Found: C, 66.17; H, 9.11; N, 5.43%, $C_{14}H_{23}NO_3$ requires C, 66.40;

H, 9.09, N, 5.53%). The crude material was found to contain a small amount of the [(1RS)-1-hydroxyethyl]-diastereoisomers.

Example 8

(5'RS,6'SR,7'RS)-5'-methyl-7'-[(1SR)-1-(p-nitrobenzyloxycarbonyloxy)-ethyl]spiro[cyclohexane-1,2'-[3]oxa[1]azabicyclo[4.2.0]octane]-8'-one

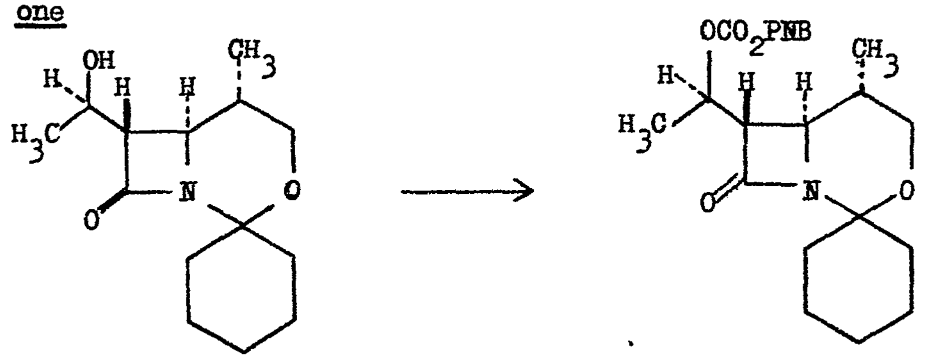

(9) (10)

A solution of (5'RS,6'SR,7'RS)-5'-methyl-7'-[(1SR)-1-hydroxyethyl]spiro[cyclohexane-1,2'-[3]oxa[1]azabicyclo[4.2.0]octane]-8'-one (9) (1.06 g) in dry tetrahydrofuran (50 ml) was cooled to -70°C and treated with n-butyl lithium (1.5 M solution in hexane; 3 ml) under an argon atmosphere. After 10 minutes, a solution of p-nitrobenzyl chloroformate (1.28 g) in tetrahydrofuran (15 ml) was added and the solution allowed to warm to room temperature over a period of 45 mins. The reaction mixture was washed with saturated ammonium chloride solution (3 x 10 ml) and the aqueous washings back-extracted into ethyl acetate. The combined organic phases were dried over anhydrous magnesium sulphate, evaporated, and the residue chromatogramed on Kieselgel 60 (<230 mesh ASTM) using 1:1 ethyl acetate/hexane as eluant, to give (5'RS,6'SR, 7'RS)-5'-methyl-7-[(1SR)-1-(p-nitrobenzyloxycarbonyloxy)-ethyl]spiro[cyclohexane-1,2'-[3]oxa[1]azabicyclo[4.2.0]octane-8'-one (10) as a white solid (1.080 g) which was recrystallised from ethyl acetate/hexane; m.p. 144 - 146°C; $\nu_{max.}$ ($CHCl_2$) 1750, 1610, 1525, 1350 $cm^{-1}$; δ (ppm., $CDCl_3$); 0.85 (3H, d, J = 7Hz, 5'$CH_3$); 1.35 - 1.87 (10 H, m, cyclohexyl and 5'-H); 1.46 (3H, d, J = 7Hz. $C\underline{H}_3$COR); 2.25 - 2.38 (1H, m, cyclohexyl); 2.92 (1H, dd, J = 7.8, 1.6 Hz, 7'-H); 3.10 (1H, dd, J = 9.8, 1.6 Hz, 6'-H); 3.49 and 3.45 (1H, ABq. J = 12 Hz, 4'-H); 3.69 and 3.67 (1H, ABq, J = 12Hz, 4'-H); 5.10 (m, appears

as 5 lines, CHCOR); 5.25 (2H, s, benzylic $CH_2$); 7.54 and 8.24 (4H, ABq, J = 9Hz, aromatic); (Found C, 61.12; H, 6.46, N, 6.52%. $C_{22}H_{28}N_2O_7$ requires C, 61.11; H, 6.48; N, 6.48%).

Example 9

(3RS, 4SR)-4-[(1RS)-2-Hydroxy-1-methylethyl]-3-[(1SR)-1-(p-nitrobenzyloxycarbonyloxy)ethyl]azetidin-2-one

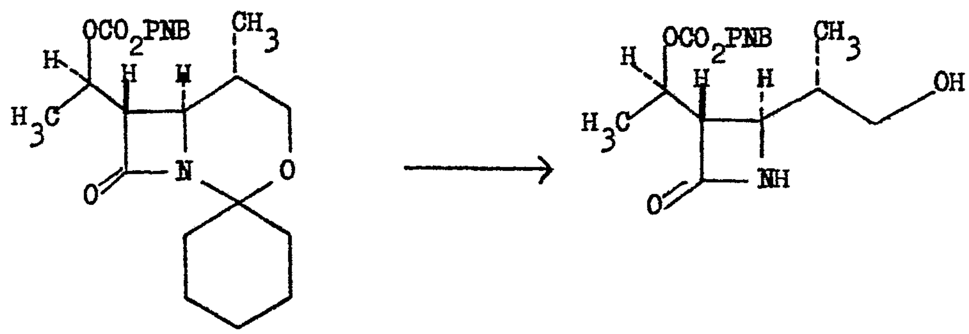

(10) (11)

A solution of (5'RS,6'SR,7'RS)-5'-methyl-7'-[(1SR)-1-(p-nitro benzyloxycarbonyloxy)ethyl]spiro[cyclohexane-1,2'-[3]oxa[1]azabicyclo [4.2.0]octane]-8'-one (10) (1.080 g) in dioxan (40 ml) was treated with a solution of concentrated sulphuric acid (1.65 ml) in water (16.5 ml) and heated to 50°C for 3 h, and for a further hour at 80°C. The solution was cooled, neutralized with saturated sodium bicarbonate and concentrated until turbid. The product was extracted into ethyl acetate (3 x 70 ml), the combined organic extracts dried over anhydrous magnesium sulphate and evaporated. Chromatography of the residue on Kieselgel 60 (230 - 400 mesh ASTM) eluting with ethyl acetate grading to 9:1 ethyl acetate/ethanol gave (3RS,4SR)-4-[(1RS)-2-hydroxy-1-methylethyl[-3-[(1SR)-1-(p-nitrobenzyloxycarbonyloxy) ethyl]azetidin-2-one (11) as a ~~white~~ foam (0.66 g); $\nu_{max.}$ ($CH_2Cl_2$) 3420, 3400 br, 1750, 1610, 1525, 1380, 1350$cm^{-1}$; δ(ppm, $CDCl_3$); 0.88(3H, d, J = 7Hz, $\underline{CH_3}CH<$); 1.48(3H, d, J = 7Hz, $\underline{CH_3}CH(OR)$); 1.77 - 2.00(2H, br.s. O-H and $CH_3\underline{CH}<$); 3.04(1H,ddd, J = 1.5, 2, 7.5 Hz, 3-H); 3.44 (1H, dd, J = 2,8.5 Hz, 4-H); 3.48(1H, dd, J = 8, 10Hz, C$\underline{Ha}$HbOH); 3.73(1H, dd, J=5,10Hz, CHa$\underline{Hb}$OH); 5.08 - 5.18 (1H, m, appears as 6 lines $CH_3\underline{CH}(OR)$); 5.25(2H, s, benzylic $\underline{CH_2}$); 5.25 (2H, s, benzylic $\underline{CH_2}$); 7.54 and 8.25(4H, ABq, J = 9Hz, aromatic).

Example 10

Methyl(4RS)-(E)-4-[(3SR,4RS)-3-[(1RS)-1-(p-nitrobenzyloxycarbonyl oxy)ethyl]-2-oxoazetidin-4-yl]-pent-2-enoate

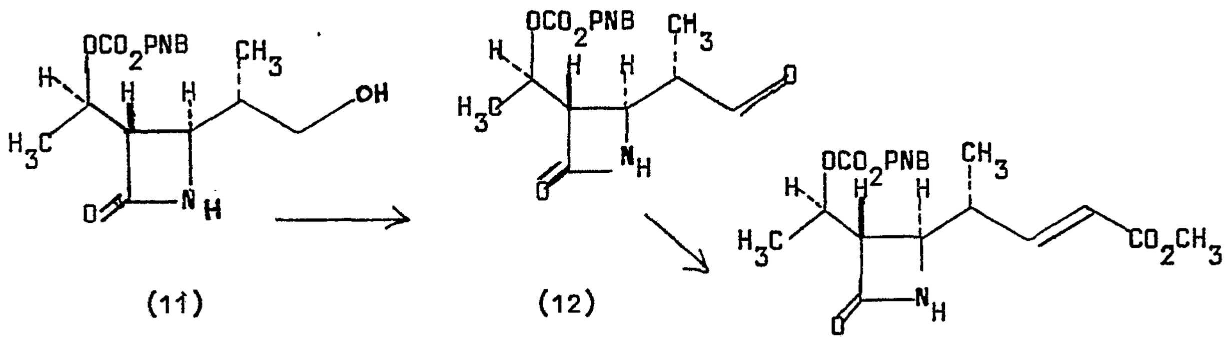

(11) (12)

(13)

A solution of (3RS,4SR)-4-[(1RS)-2-hydroxy-1-methylethyl]-3-[(1SR)-1-(p-nitrobenzyloxycarbonyloxy)ethyl]azetidin-2-one (11) (0.647 g) in toluene (6 ml) and dry dimethylsulphoxide (3 ml) was treated with 1-ethyl-3-(3-dimethylaminopropyl)carbodiimide hydrochloride (1.15 g), pyridine (0.237 g) and trifluoroacetic acid (0.114 g). The reaction mixture was stirred at room temperature for 4 hours, then partitioned between ethyl acetate and water. The aqueous phase was extracted with ethyl acetate (30 ml) and the combined organic phases washed with 2N hydrochloric acid (2 x 30 ml), saturated sodium bicarbonate solution (2 x 30 ml) and saturated brine (2 x 30 ml), then dried over anhydrous magnesium sulphate. The solution was evaporated to give (2RS)-2-[(3RS),(4SR)-3-[(1SR)-1-p-nitrobenzyloxycarbonyloxy)ethyl]-2-oxoazetidin-4-yl]-propanal (12) as a colourless oil which was not purified further, and was redissolved in toluene (10 ml). The resulting solution was treated with carbomethoxymethylene triphenyl phosphorane (0.88 g) and stirred at room temperature for 1 h. The solvent was evaporated and the residue chromatographed on Kieselgel 60 (230 - 400 mesh ASTM) eluting with 1:1 hexane/ethyl acetate, to give methyl(4RS)-(E)-4-[(3SR,4RS)-3-[(1RS)-1-(p-nitrobenzyloxycarbonyloxy)ethyl]-2-oxoazetidin-4-yl]-pent-2-enoate (13) as a

colourless oil (0.575 g); $\nu_{max.}$ ($CH_2Cl_2$ ) 3400, 1770, 1750, 1520, 1350cm$^{-1}$; δ(ppm; $CDCl_3$) 1.10 (3H, d, J = 7Hz, C$\underline{H}_3$CH<); 1.47 (3H, d, J = 7Hz, C$\underline{H}_3$CH(OR)); 2.37 - 2.52 (1H, m, appears as 6 lines, $CH_3$C$\underline{H}$ ); 3.03 (1H, dd, J = 7.8, 2.3Hz, 3-H); 3.49 (1H, dd, J = 8.6, 2.3Hz, 4-H ); 3.75 (3H, s, $CO_2$C$\underline{H}_3$); 5.08 - 5.18 (m, appears as 5 lines, $CH_3$C$\underline{H}$(OR)); 5.25 (2H, s, C$\underline{H}_2$ benzyl); 5.89 (1H, dd, J = 15.4, 0.8 Hz, C$\underline{H}$-$CO_2CH_3$); 5.91 (1H, br.s., N-H); 6.78 (1H, dd, J = 15.4 8.6 Hz, C$\underline{H}$ = CH-$CO_2CH_3$); 7.54 and 8.25 (4H, ABq, J = 9Hz, aromatic).

Example 11

p-Nitrobenzyl 2-[(3RS,4SR)-4-[(1SR)-(E)-3-methoxycarbonyl-1-methyl-prop-2-enyl]-3-[(1SR)-1-p-nitrobenzyloxycarbonyloxy)ethyl]-2-oxo-azetidin-1-yl]-2-hydroxyacetate

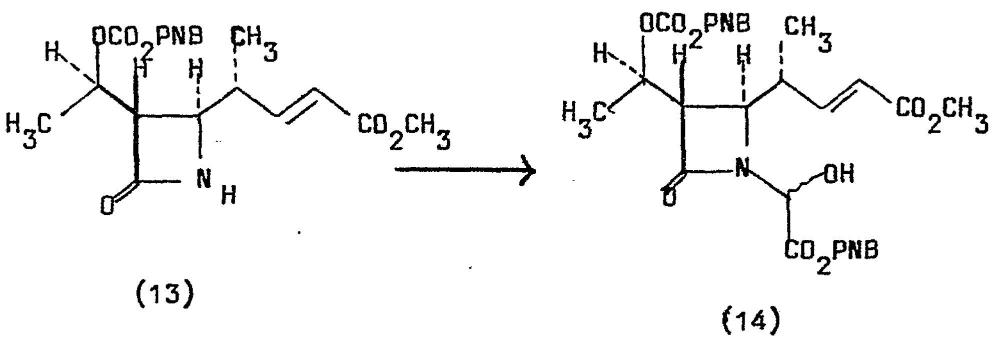

(13)

(14)

A suspension of p-nitrobenzyl glyoxalate monohydrate (13) (0.77 g) was heated to reflux in benzene (35 ml) in a Dean and Stark apparatus for 1 hr. to remove water. To the resulting clear solution was added methyl (4RS)-(E)-4-[(3SR,4RS)-3-[(1RS)-1-(p-nitrobenzyloxy carbonyloxy)ethyl]-2-oxo-azetidin-4-yl]-pent-2-enoate (0.570 g) and the mixture heated to reflux under an argon atmosphere for 10 hours. The solvent was evaporated and the residue chromatographed on Kieselgel 60 (<230 mesh ASTM) using 1:1 hexane/ethyl acetate as eluant to give p-nitrobenzyl 2-[(3RS,4SR)-4-[(1SR)-(E)-3-methoxycarbonyl-1-methyl-prop-2-enyl]-3-[(1SR)-1-p-nitrobenzyloxycarbonyloxy) ethyl-2-oxo-azetidin-1-yl]-2-hydroxyacetate (14), a mixture of two diastereoisomers, as a pale yellow oil which was contaminated with p-nitrobenzyl glyoxalate in a molar ratio of 2:1 product: p-nitrobenzyl glyoxalate (total weight 1.06 g); $\nu_{max.}$ ($CH_2Cl_2$) 3500, 1770, 1750, 1735, 1610, 1525, 1375, 1350 $cm^{-1}$; $\delta$(ppm, $CDCl_3$., major isomer, inter alia) 1.08 (3H, d, J = 7Hz, $C\underline{H}_3CH$ ); 1.40 (3H, d, J = 7Hz, $C\underline{H}_3CH(OR)$; 2.57 - 2.68 (1H, m, $CH_3C\underline{H}$); 3.06 (1H, dd, J = 8, 2 Hz, 3-H); 3.71 (3H, s, $CO_2C\underline{H}_3$); 3.82 (1H, dd, J = 9,2Hz, 4-H); 5.90 (1H, d, J = 15Hz, $C\underline{H}-CO_2CH_3$); 6.84 (1H, dd, J = 15, 9Hz, $C\underline{H}=CH-CO_2CH_3$); 7.50 - 7.65 (4H, m, aromatic); 8.23 - 8.29 (4H, m, aromatic);

δ (ppm, $CDCl_3$, minor isomer, *inter alia*) 1.09 (3H, d, J = 7Hz, $C\underline{H}_3CH$); 1.42 (3H, d, J = 7Hz, $C\underline{H}_3CH(OR)$); 2.57 - 2.68 (1H, m, $CH_3C\underline{H}$); 3.05 - 3.12 (1H, m, 3-H); 3.71 (1H, s, $COC\underline{H}_3$); 3.80 - 3.86 (1H, m, 4-H); 5.87 (1H, d, J = 15Hz, $C\underline{H}CO_2CH_3$); 6.78 (1H, dd, J =15, 9Hz, $C\underline{H}$ = $CH-CO_2CH_3$); 7.5 - 7.65 (4H, m, aromatic) 7.23 - 8.29 (4H, m, aromatic).

Example 12

p-Nitrobenzyl 2-[(3RS,4SR)-4-[(1SR)-(E)-3-methoxycarbonyl-1-methyl-prop-2-enyl]-3-[(1SR)-1-(p-nitrobenzyloxycarbonyloxy)ethyl]-2-oxo-azetidin-1-yl]-2-(triphenylphosphoranylidene)acetate

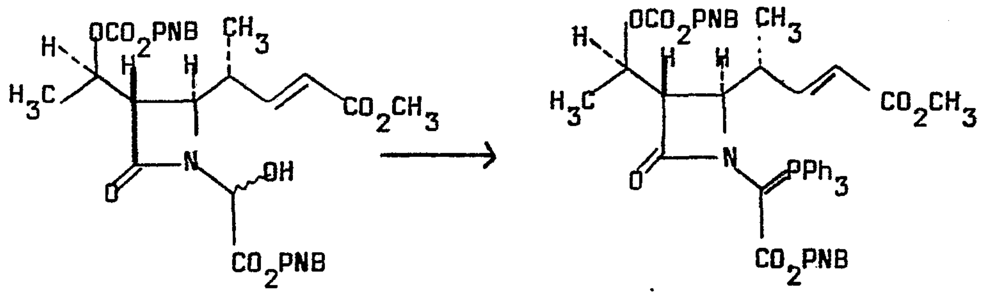

(14) (15)

The diastereoisomeric mixture of p-nitrobenzyl 2-[(3RS,4SR)-4-[(1SR)-(E)-3-methoxycarbonyl-1-methyl-prop-2-enyl]-3-[(1SR)-1-p-nitrobenzyloxycarbonyloxy)ethyl]-2-oxo-azetidin-1-yl-2-hydroxyacetates (14) contaminated with p-nitrobenzyl glyoxalate (total weight 1.000 g) was dissolved in dry tetrahydrofuran (20 ml), cooled to -20°C, and treated with 2,6-lutidine (0.53 ml) followed by thionyl chloride (0.33 ml). The mixture was stirred at -20°C for 30 minutes, then filtered and evaporated to a gum which was re-evaporated twice from toluene. The residue, a yellow oil, was dissolved in dioxan (20 ml) and treated with 2,6-lutidine (0.53 ml) and triphenyl phosphine (1.165 g). The mixture was stirred at room temperature for 16 hours then at 60°C for a further 4 hours to complete reaction. This was filtered, the solvent evaporated, and the residue chromatographed on Kieselgel 60 (<230 mesh ASTM) using 1:1 ethylacetate/hexane as eluant to give p-nitrobenzyl 2-[(3RS,4SR)-4-[(1SR)-(E)-3-methoxycarbonyl-1-methyl-prop-2-enyl]-3-[(1SR)-1-(p-nitrobenzyloxycarbonyloxy)ethyl]-2-oxo-azetidin-1-yl]-2-(triphenylphosphoranylidene)acetate (15) (0.602 g) as a pale yellow foam; $\nu_{max.}$ ($CH_2Cl_2$) 1745, 1720 (sh), 1610, 1525, 1350$cm^{-1}$.

Example 13

(2RS)-2-[(3RS,4RS)-3[(1SR)-1-(p-nitrobenzyloxycarbonyloxy)ethyl]-1-[(p-nitrobenzyloxycarbonyl)(triphenyl phosphoranylidene)methyl]-2-oxoazetidin-4-yl]-propanoic acid

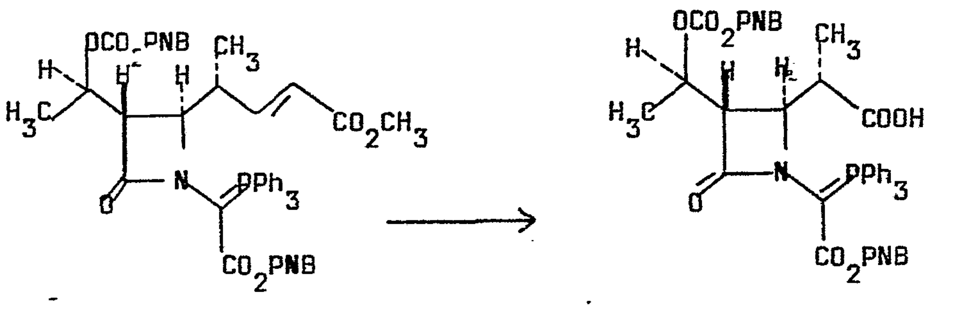

(15) (16)

p-Nitrobenzyl 2-[(3RS,4SR)-4-[(1SR)-(E)-3-methoxycarbonyl-1-methylprop-2-enyl]-3-[(1SR)-1-(p-nitrobenzyloxycarbonyloxy)ethyl]-2-oxo-azetidin-1-yl]-2-(triphenylphosphoranylidene)acetate (15) (0.602 g) was dissolved in dry dichloromethane (30 ml), cooled to 0°C and treated with trifuloroacetic acid (3 ml). After 15 minutes the solution was cooled to -50°C, and ozone passed through until the colour just turned blue. Argon was blown through to remove excess ozone, and the solution treated with m-chloroperbenzoic acid (0.133 g) in dry dichloromethane ( 2 ml). The reaction mixture was stirred for 20 hours at room temperature, then evaporated and the residue re-evaporated twice from toluene to remove excess trifluoroacetic acid. The crude product was chromatographed on Kieselgel 60 (240 - 400 mesh ASTM) using 4:1 ethyl acetate/hexane as eluant to give (2RS)-2-[(3RS, 4RS)-3-[(1SR)-1-(p-nitrobenzyloxycarbonyloxy)ethyl]-1-[(p-nitrobenzyl oxycarbonyl)(triphenylphosphoranylidene )methyl]-2-oxoazetidin-4-yl]-propanoic acid (16) (0.316 g); $\nu_{max.}$ ($CH_2Cl_2$) 1745, 1610, 1520, 1350 $cm^{-1}$.

Example 14

p-Nitrobenzyl 2-[(3RS,4RS)-3-[(1SR)-1-(p-nitrobenzyloxycarbonyloxy) ethyl-2-oxo-4-[(1RS)-1-(pyrimidin-2-yl thiocarbonyl)-1-ethyl]-azetidin-1-yl]-2-(triphenylphosphoranylidene)acetate

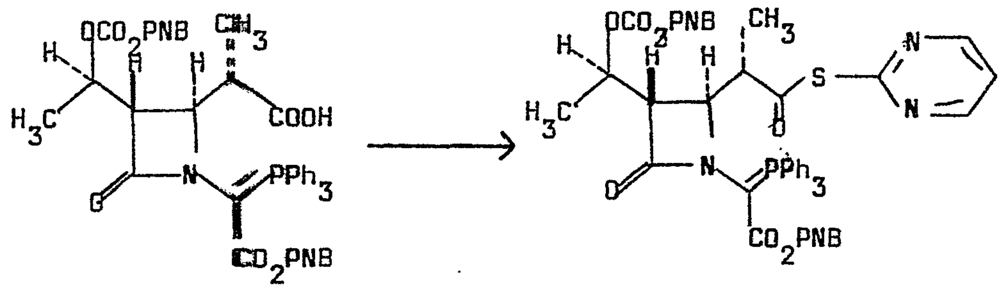

(16) (17)

Pyrimidine-2-thiol (0.062 g) was added to a stirred solution of lithium (0.004 g) in ethanol (2 ml). The thiol dissolved and the resultant solution was evaporated and re-evaporated twice from toluene to give lithium pyrimidine-2-thiolate as a yellow solid. (2RS)-2-[(3RS,4RS)-3-[(1SR)-1-(p-nitrobenzyloxycarbonyloxy)ethyl]-1-[(p-nitrobenzyloxycarbonyl)(triphenylphosphoranylidene)methyl]-2-oxoazetidin-4-yl]propanoic acid (16) (0.316 g) was dissolved in dry tetrahydrofuran (10 ml), treated with triethylamine (0.055 g) and diethylchlorophosphate (0.090 g) and the reaction mixture stirred at room temperature under an argon atmosphere for 2.5 hours. The reaction mixture was then treated with the freshly-prepared lithium pyrimidine-2-thiolate (0.066 g) as a solid and stirring continued for a further 3 hours. The mixture was filtered, the solvent evaporated and the residue chromatographed on Kieselgel 60 (<230 mesh ASTM) using ethyl acetate as eluant to give p-nitrobenzyl 2-[(3RS,4RS)-3-[(1SR)-1-(p-nitrobenzyl oxycarbonyloxy)ethyl-2-oxo-4-[(1RS)-1-(pyrimidin-2-yl thiocarbonyl)-1-ethyl]-azetidin-1-yl]-2-(triphenyl phosphoranylidene)acetate as a pale yellow foam (17) (0.228 g); $\nu_{max.}$ ($CH_2Cl_2$) 1745, 1720(sh), 1610, 1550, 1525, 1380, 1350 $cm^{-1}$.

Example 15

p-Nitrobenzyl (4RS,5RS,6RS)-4-methyl-6-[(1SR)-1-(p-nitrobenzyloxycarbonyloxy)ethyl]-3-(pyrimidin-2-ylthio)-7-oxo-1-azabicyclo[3.2.0]hept-2-ene-2-carboxylate

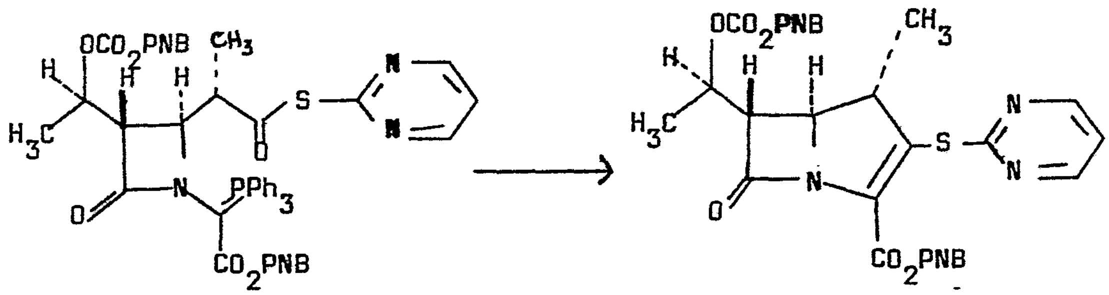

(17)                                                  (18)

p-Nitrobenzyl 2-[(3RS,4RS)-3-[(1SR)-1-(p-nitrobenzyloxycarbonyloxy)ethyl-2-oxo-4-[(1RS)-1-(pyrimidin-2-ylthiocarbonyl)-1-ethyl]-azetidin-1-yl]-2-triphenylphosphoranylidene)acetate (17) (0.220 g) was dissolved in dry toluene (200 ml) and heated to reflux in a Dean and Stark apparatus under an argon atmopshere for 4 hours. The resulting solution was concentrated under reduced pressure and chromatographed on Kieselgel 60 (<230 mesh ASTM) using 7:3 ethyl acetate/hexane as eluant, to afford p-nitrobenzyl (4RS,5RS,6RS)-4-methyl-6[(1SR)-1-p-nitrobenzyloxycarbonyloxy)ethyl]-3-(pyrimidin-2-yl thio)-7-oxo-1-azabicyclo[3.2.0]hept-2-ene-2-carboxylate (18) (0.090 g) as a pale yellow foam which was crystallised from ethyl acetate, m.p. 144 - 146°C, $\nu_{max.}$ ($CH_2Cl_2$) 1785, 1750, 1730, 1610, 1560, 1525, 1380, 1350cm$^{-1}$; $\delta$(ppm, $CDCl_3$) 1.15 (3H, d, J = 7Hz, 4-$CH_3$); 1.54 (3H, d, J = 6.3 Hz, 8-$CH_3$); 3.57 (1H, dd, J = 3.2, 7.4 Hz, 6-H); 3.90 (1H, dd, J = 8.2, 3.2, Hz, 5-H); 3.96 - 4.07 (1H, m, appears as 5 lines, 4-H); 5.15 - 5.26 (1H, m, 8-H); 5.26 (2H, s, $CH_2$ benzyl); 5.27 and 5.45 (2H, ABq, J = 15 Hz, $CH_2$ benzyl); 7.05 (1H, t, J = 5Hz, pyrimidinyl-H); 7.54, 7.62, 8.20 and 8.22 (4H, 2 x ABq, J = 9Hz, nitrophenyls); 8.53 (2H, d, J = 5Hz, pyrimidinyl); (Found: C, 54.56; H, 3.88; N, 11.05%, $C_{29}H_{25}N_5O_{10}S$ requires C, 54.80 H, 3.94; N, 11.02%).

Example 16

Sodium (4RS,5RS,6RS)-4-methyl-6-[(1SR)-1-hydroxyethyl]-3-(pyrimidin-2-ylthio)-7-oxo-1-azabicyclo[3.2.0]hept-2-ene-2-carboxylate

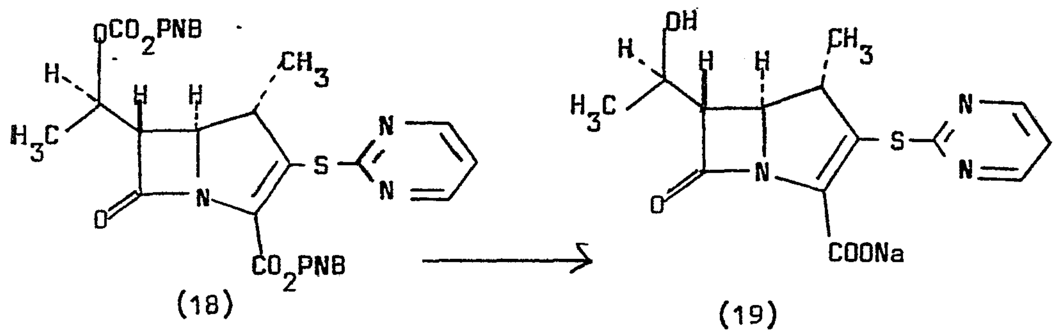

(18) (19)

p-Nitrobenzyl (4RS,5RS,6RS)-4-methyl-6-[(1SR)-1-(p-nitrobenzyloxycarbonyloxy)ethyl]-3-(pyrimidin-2-ylthio)-7-oxo-1-azabicyclo-[3.2.0]hept-2-ene-2-carboxylate (18) (0.040 g) was dissolved in 20% aqueous dioxan (20 ml) containing pH 7 phosphate buffer (0.05 M; 4 ml) and 10% palladium-charcoal catalyst (pre-hydrogenated in the solvent for 25 minutes, 0.025 g), and was hydrogenated at ambient temperature and pressure for 2 hours. The resultant mixture was treated with a solution of sodium bicarbonate (0.006 g) in water (2 ml), filtered through Kieselguhr, the organic solvent evaporated and the aqueous solution washed with ethyl acetate (3 x 10 ml). Examination of the aqueous phase by U.V. showed it to contain the title compound, sodium (4RS,5RS,6RS)-4-methyl-6-[(1SR)-1-hydroxyethyl]-3-(pyrimidin-2-ylthio)-7-oxo-1-azabicyclo[3.2.0]hept-2-ene-2-carboxylate (19) (0.011 g); $\lambda_{max}$ ($H_2O$) 292.5, 246.5 n.m.

DEMONSTRATION OF EFFECTIVENESS

In a standard MIC test the following data were obtained.

| ORGANISM | MIC (µg/ml) |
|---|---|
| Escherichia coli 0111 | 1.6 |
| Salmonella typhimurium CT10 | 1.6 |
| Staphylococcus aureus Russell | 0.4 |

Example 17

Resolution of 3-amino-2-methyl propan-1-ol

To a solution of racemic 3-amino-2-methyl propan-1-ol (4) (3.0 g) in methyl ethyl ketone (65 ml) was added (-) N-(1-phenylethyl)-succinamic acid (7.6 g) (E. Felder, D. Pitre and S. Boveri, Helv. Chim. Acta, 1969, 52, 329) at 60°C and the mixture stirred until dissolved. On cooling the stirred solution to ambient temperature, the desired salt was precipitated. This was filtered, washed with ether and dried in vacuo to yield 3.7 g of crystalline material. On recrystallisation from methylethyl ketone the optical rotation remained constant; $[\alpha]_D^{20}$ = -64.2° (C = 1, ethanol); m.p. 112-115°C; Found: C, 61.59; H, 8.29; N, 8.95%, $C_{16}H_{26}N_2O_4$ requires C, 61.94; H, 8.39; N, 9.03%.

A solution of the salt in methanol was applied to strongly basic ion-exchange column ("Amberlyst" A-26 resin) Elution with methanol followed by evaporation of solvent gave the optically active amino alcohol; $[\alpha]_D^{20}$ = +7.8° (C = 1.7, chloroform).

In a similar manner, the (-) amino alcohol was prepared by resolution using (+) N-(1-phenylethyl)-succinamic acid.

Example 18

1-[(S)-3-methyl-1-oxa-5-azaspiro [5.5] undecan-5-yl] butan-1, 3-dione

The salt of (+) -(S)-3-amino-2-methyl propan-1-ol with (-) -N-(1-phenylethyl)-succinamic acid was prepared as described in example 17. This gave a rotation of -67.8° (c1, ethanol).

The salt (41.0 g) was suspended in dry benzene (500 ml), treated with triethylamine (13.3 g) and cyclohexanone (14.8 g) and the mixture heated to reflux in an apparatus with provision for water removal for 5 hours. The solution was cooled to 0°C, filtered, and the filtrate treated with diketene (9.6 ml) at 0°C under an argon atmosphere and the mixture stirred at 0°C for 1.5 hours.

The solvent was evaporated and the residue chromatographed on Kieselgel 60 (<230 mesh ASTM) eluting with hexane grading to 1:1 ethyl acetate/hexane to give the title compound (23.2 g, 70%), $[\alpha]^{20}$ = -22.1° (c1, chloroform).

Example 19

1-[(S)-3-Methyl-1-oxa-5-azaspiro [5.5] undecan-5-yl]-2-diazabutan-1, 3-dione

1-[(S)-3-Methyl-1-oxa-5-azaspiro [5.5] undecan-5-yl] butan-1, 3-dione was converted into the title compound in 65% yield on treatment with methane sulphonyl azide under the conditions described in Example 5.

Example 20

(5'S,6'R,7'S)-7'-Acetyl-5'-methylspiro[cyclohexane-1,2'-[3]oxa[1]azabicyclo [4.2.0]octane]-8'-one

Cyclisation of 1-[(S)-3-Methyl-1-oxa-5-azaspiro [5.5] undecan-5-yl]-2-diazabutan-1,3-dione under the conditions described in Example 6 took place in 64% yield. The product was a mixture of epimers at C-5' of which 88% was the title compound. This was obtained pure by column chromatography, $[\alpha]^{20}_{D} = +4.5^{o}$ (c2, chloroform).

Example 21

(5'S,6'R,7'S)-5'Methyl-7'-[(R)-1-hydroxyethyl] spiro [cyclohexane-1,2'-[3]oxa[1]azabicyclo [4.2.0] octane-8'-one

(5'S,6'R,7'S)-7'-Acetyl-5'-methylspiro [cyclohexane-1,2'-[3]oxa[1]azabicyclo [4.2.0] octane]-8'-one was reduced with potassium tri-sec-butyl borohydride under the conditions described in Example 7. The desired product, (5'S,6'R,7'S)-5'-Methyl-7'-[(R)-1-hydroxyethyl]spiro[cyclohexane-1,2'-[3]oxa[1] azabicyclo [4.2.0] octane]-8'-one was crystallised from ethyl acetate/hexane, $[\alpha]^{20}$ = -36.3% (cl, chloroform).

0089139

Example 22

(5'S,6'R,7'S)-5'-Methyl-7'[(R)-1-(p-nitrobenzyloxycarbonyloxy)-ethyl]spiro[cyclohexane-1,2'-[3]oxa[1]azabicyclo [4.2.0] octane]-8'-one

(5'S,6'R,7'S)-5'-Methyl-7'-[(R)-1-hydroxyethyl] spiro[cyclohexane-1,2'-[3]oxa[1]azabicyclo [4.2.0] octane]-8'-one (2.70 g) was dissolved in dry tetrahydrofuran (10 ml) and added to a solution of lithium diisopropylamide (diisopropylamine (1.04 g) and n-butyl lithium (1.55M, 6.7 ml)) at -60°C.

The solution was stirred at -60°C for 15 minutes, then treated with a solution of p-nitrobenzyl chloroformate (2.45 g) in the THF (8 ml) and allowed to warm to room temperature over a period of 1 hour. The reaction mixture was worked up as described in Example 8 to yield the title product as a white foam (3.55 g, 77%), $[\alpha]^{20}$ = -25.9° (c2, chloroform).

Example 23

(3S,4R)-4-[(1S)-2-Hydroxy-1-methylethyl]-3-[(1R)-1-(p-nitrobenzyloxycarbonyloxy)ethyl] azetidin-2-one.

(5'S, 6'R, 7'S)-5'-Methyl-7'-[(R)-1-(p-nitrobenzyloxy carbonyloxy)-ethyl]spiro [cyclohexane-1,2'-[3]oxa[1] azabicyclo [4.2.0] octane]-8'-one (3.51g) was treated with concentrated sulphuric acid under the conditions described in Example 9, to yield the title compound (2.42g, 78%) which crystallised from ethylacetate/hexane. $[\alpha]^{20} = -16.0^{o}$ (c1.1,chloroform).

0089139

Example 24

Methyl (4R)-(E)-4-[(3S,4R)-3-[(1R)-1-(p-nitrobenzyl oxycarbonyloxy)ethyl]-2-oxoazetidin-4-yl] pent-2-enoate.

(3S,4R)-4-[1S)-2-Hydroxy-1-methylethyl]-3-[(1R)-1-(p-nitrobenzyloxycarbonyloxy) ethyl] azetidin-2-one (2.24g) was oxidised under modified Moffat conditions and the intermediate aldehyde trapped with carbomethoxymethylene triphenyl phosphorane, as described in Example 10. The title product was obtained as a white foam (1.95g, 75%), $[\alpha]^{20} = +17.95^{o}$ (c1.6,chloroform).

Example 25

p-Nitrobenzyl 2-[(3S,4R)-4-[(1R)-(E)-3-methoxycarbonyl-1-methyl-prop-2-enyl]-3-[(1R)-1-p-nitrobenzyloxy carbonyl oxy)ethyl]-2-oxo-azetidin-1-yl]-2-hydroxy acetate.

Methyl (4R)-(E)-4-[(3S,4R)-3-[(1R)-1-(p-nitrobenzyl oxycarbonyloxy)ethyl]-2-oxoazetidin-4-yl] pent-2-enoate (1.95g) was dissolved in dry dimethylformamide (8ml) and treated with glyoxylic acid hydrate (0.482g) in the presence of 3A molecular sieves (1.5g). The reaction mixture was stirred at room temperature for 5 hours then treated with potassium carbonate (0.730g) and p-nitrobenzylbromide (1.14g) and stirred for a further 16 hours. The mixture was filtered, the solution partitioned between water and ethyl acetate and the organic phase washed with sodium bicarbonate solution (3x50ml), dried over anhydrous magnesium sulphate and evaporated under reduced pressure. The residue was chromatographed on Kieselgel 60 (<230 mesh ASTM) eluting with 1:1 hexane/ethyl acetate to afford the title product as a white foam (1.54g).

Example 26

p-Nitrobenzyl 2-[(3S,4R)-4-[(1R)-(E)-3-methoxycarbonyl-1-methylprop-2-enyl]-3-[(1R)-1-(p-nitrobenzyloxycarbonyloxy)ethyl]-2-oxoazetidin-1-yl]-2-(triphenylphosphoranylidene) acetate.

p-Nitrobenzyl 2-[(3S,4R)-4-[(1R)-(E)-3-methoxycarbonyl-1-methyl-prop-2-enyl]-3-[(1R)-1-p-nitrobenzyloxy carbonyloxy)ethyl]-2-oxo-azetidin-1-yl]-2-hydroxy acetate (1.54g) was converted to the title compound under the conditions described in Example 12. The title compound was obtained as a pale yellow foam (1.61g, 75%), $[\alpha]^{20}_{D} = -7.1^{0}$ (cl, chloroform).

Example 27

(2S)-2-[(3S,4S)-3[(1R)-1-(p-Nitrobenzyloxycarbonyloxy) ethyl]-1-[(p-nitrobenzyloxycarbonyl)(triphenylphosphos phoranylidene) methyl]-2-oxoazetidin-4-yl] propanoic acid

p-Nitrobenzyl 2-[(3S,4R)-4-[(1R)-[E]-3-methoxycarbonyl-1-methylprop-2-enyl]-3-[(1R)-1-(p-nitrobenzyl oxycarbonyloxy) ethyl]-2-oxo-azetidin-1-yl]-2-(triphenylphosphoranylidene) acetate (1.60g) was converted to the title compound (0.600g) under the conditions described for Example 13. The desired product was obtained as a pale yellow foam $[\alpha]^{20}$ = $-11.5^0$ (cl, chloroform).

0089139

Example 28

p-Nitrobenzyl 2-[(3S,4S)-3-[(1R)-1-(p-nitrobenzyl oxycarbonyloxy) ethyl]-2-oxo-4-[(1S)-1-(pyrimidin-2-yl thiocarbonyl)-1-ethyl]-azetidin-1-yl]-2-(triphenyl phosphoranylidene) acetate.

(2S)-2-[(3S,4S)-3[(1R)-1-(p-Nitrobenzyloxycarbonloxy) ethyl]-1-[(p-nitrobenzyloxycarbonyl) (triphenyl phosphoranylidene)methyl]-2-oxoazetidin-4-yl]-propanoic acid (0.600g) was converted to the thio-ester under the conditions described in Example 14. The title compound was obtained as a pale yellow foam (0.534g, 80%) $[\alpha]^{20}$ = +19.1$^{0}$ (cl, Chloroform).

Example 29

p-Nitrobenzyl (4S, 5S, 6S)-4-methyl-6-[(1R)-1- (p-nitro benyloxycarbonyloxy)ethyl]-3-(pyrimindin-2-yl thio)-7-oxo-1-azabicyclo [3.2.0] hept-2-ene-2-carboxylate

The title compound was obtained in 65% yield from p-nitrobenzyl 2-[(3S,4S)-3-[(1R)-1-(p-nitrobenzyloxy carbonyloxy)ethyl-2-oxo-4-[(1S)-1-(pyrimidin-2-yl thiocarbonyl)-1-ethyl]-azetidin-1-yl]-2-(triphenyl phosphoranylidene) acetate under the conditions described for example 15. $[\alpha]_D^{20} = -138.3^0$ (c1, Chloroform).

Example 30

Sodium (4S,5S,6S)-4-methyl-6-[(1R)-1-hydroxyethyl]-3-(pyrimidin-2-ylthio)-7-oxo-1-azabicyclo [3.2.0] hept-2-ene-2-carboxylate.

Hydrogenolysis of p-nitrobenzyl (4S,5S,6S)-4-methyl-6-[(1R)-1-(p-nitrobenzyloxycarbonyloxy)ethyl]-3-(pyrimidin-2-yl thio)-7-oxo-1-azabicyclo [3.2.0] hept-2-ene-2-carboxylate (0.020g) under the conditions described in example 16, gave the title compound (0.006g). This demonstrated activity against a variety of organisms. Representative biological data is as follows:

| ORGANISM | M.I.C(μg/ml) |
|---|---|
| E.coli JT39 | 0.8 |
| S.typhimurium CT10 | 3.1 |
| S.aureus Russell | 0.8 |

Example 31

(5'RS,6'RS,7'SR)-5'-Methyl-7'-[1(RS,SR)-1-hydroxyethyl] spiro[cyclohexane-1,2'-[3]oxa[1]azabicyclo [4.2.0] octane]-8'-one

(5'RS,6'RS,7'SR)-7'-Acetyl-5'-methyl spiro [cyclohexane-1,2'-[3]oxa[1]azabicyclo [4.2.0] octane]-8'-one (1.89 g), prepared by the method of Example 6, was treated with potassium tri-sec-butyl borohydride under the conditions described in Example 7. After work-up and chromatography, the title compound was obtained as a colourless foam as a mixture of (RS) and (SR) isomers in the hydroxyethyl side-chain in a 4:1 ratio (1.20 g); δppm, $CDCl_3$, major isomer) 1.13 (3H, d, J=6Hz, 5' $CH_3$); 1.30 (3H, d, J=6Hz, $C(O)CH_3$); 1.35-2.00 (10H, m, cyclohexyl and 5'-H); 2.14 (1H, d, O-H); 2.25-2.35 (1H, m, cyclohexyl); 3.04 (1H, dd, J=6.6, 2.0Hz, 7'-H); 3.55 + 3.59 (1H, ABq, J=3.0Hz, 4'-H); 3.75 (1H, dd, J=2.0, 5.1 Hz, 6'-H); 3.94 and 3.98 (1H, ABq, J=3.0Hz, 4'-H); 4.08 - 4.20 (1H, m, CHOH).

## Example 32

(5'RS,6'RS,7'SR)-5'-Methyl-7'-[1,(RS,SR)-1-(p-nitrobenzyloxycarbonyloxy)-ethyl]spiro[cyclohexane-1,2'-[3]oxa[1]azabicyclo [4.2.0]octane]-8'-one

The product from Example 31 (1.20g) was converted to the title compound in 76% yield following the procedure of Example 8. The 4:1 ratio of (RS) : (SR) p-nitrobenzyloxycarbonyloxyethyl isomers was retained. νmax ($CH_2Cl_2$) 1745, 1610, 1525, 1450, 1375, 1350 $cm^{-1}$; δ(ppm, $CDCl_3$, major isomer) 1.12 (3H, d, J=7Hz, 5'-$CH_3$); 1.35-1.97 (10H, m, cyclohexyl and 5'-H); 1.45 (3H, d, J=7Hz, C<u>H</u>$_3$COR); 2.25-2.38 (1H, m, cyclohexyl; 3.17 (1H, dd, J=2.0, 7.5Hz, 7'-H); 3.54 and 3.58 (1H, ABq, J=3.0 Hz, 4'-H); 3.70 (1H, dd, J=2.0, 5.5Hz, 6'-H); 3.92 and 3.98 (1H, ABq, J=3.0Hz, 4'-H); 5.00 - 5.15 (1H, m, appears as 5 lines, C<u>H</u>COR); 5.25 (2H, s, benzylic $CH_2$); 7.54 and 8.24 (4H, ABq, J=9Hz aromatic).

Example 33

(3RS,4SR)-4-[(1SR)-2-Hydroxy-1-methylethyl]-3-[(1SR)-1-(p-nitrobenzyloxycarbonyloxy)ethyl] azetidin-2-one

The product from Example 32 (1.42 g) was treated with concentrated sulphuric acid under the conditions described in Example 9. The product was obtained as a mixture of p-nitrobenzyloxycarbonyloxyethyl isomers (1.000g, 86%) from which the title compound was obtained by crystallisation from ethyl acetate/hexane; νmax ($CH_2Cl_2$); 3600, 3400, 1765, 1610, 1525, 1380, 1350 $cm^{-1}$; δ(ppm, $CDCl_3$); 1.00 (3H, d, J=7Hz, $C\underline{H}_3CH<$); 1.49 (3H, d, J=7Hz, $C\underline{H}_3CH(OR)$); 1.67 (1H, t, J=5Hz, O-H); 1.78 - 1.90 (1H, m, $CH_3CH<$); 3.20 (1H, ddd, J=0.7, 2.2, 8.2 Hz, 3-H); 3.54 - 3.69 (3H, m, 4-H and $CH_2OH$); 5.06 -5.18 (1H, m, appears as 6 lines $CH_3C\underline{H}(OR)$); 5.25 (2H, s, benzylic $C\underline{H}_2$); 5.99 (1H, br.s. N-H); 7.55 and 8.25 (4H, ABq, Ar-H).

Example 34

Methyl(4RS)-(E)-4-[(3RS,4SR)-3-[(1SR,RS)-1-(p-nitrobenzyloxycarbonyloxy)ethyl]-2-oxoazetidin-4-yl]pent-2-enoate

The product from Example 33 (0.990 g as a 4:1 mixture of p-nitrobenzyloxycarbonyloxyethyl isomers) was converted into the title compound following the procedure outlined in Example 10, in 62% yield. νmax ($CH_2Cl_2$) 3420, 1760, 1720(Sh), 1640 1610, 1525, 1350 $cm^{-1}$; δ(ppm, $CDCl_3$, major isomer); 1.13 (3H, d, J=6.2 Hz, $CH_3CH$ ); 1.42 (3H, d, J=6.5 Hz, $CH_3CH(OR)$); 2.45 - 2.60 (1H, m, appears as 5 lines $CH_3CH$ ); 3.02 (1H, dd, J=7.5, 2.2Hz, 3-H); 3.58 (1H, dd, J=7.6, 2.2Hz, 4-H); 3.70 (3H, s, $CO_2CH_3$); 5.08 -5.18 (1H, m, $CH_3CH(OR)$); 5.24 (2H, d, J=4.0Hz, $CH_2$ benzyl); 5.91 (1H, dd, J=1.1, 15.7Hz, CH $CO_2$ $CH_3$); 6.07 (1H, br.s., N-H); 6.84 (1H, dd, J=7.9, 15.7Hz, CH = $CHCOCH_3$); 7.56 and 8.25 (4H, ABq, J=9Hz, aromatic).

Example 35

p-Nitrobenzyl 2-[(3RS,4SR)-4-[(1RS)-(E)-3-methoxy carbonyl-1-methyl-prop-2-enyl]-3[(1SR)-1-p-nitro benzyloxycarbonyloxy)ethyl]-2-oxo-azetidin-1-yl]-2-hydroxyacetate.

The product from Example 34 (as a mixture of p-nitrobenzyloxycarbonyloxyethyl isomers) (0.707g) was dissolved in dry DMF (5ml) and treated with glyoxylic acid hydrate (0.200g) in the presence of 3A molecular sieves (0.50g). The reaction was stirred at room temperature for 100 hours. The resulting solution was treated with potassium carbonate (0.390g) and p-nitrobenzyl bromide (0.600g) and stirring continued at room temperature for 6 hours. The solvent was evaporated and the residue chromatographed on Kieselgel 60 (<230 mesh ASTM) using 1:1 hexane/ethyl acetate to effect separation of the p-nitrobenzyloxycarbonyl oxyethyl diastereoisomers and to afford the title product as a white foam (0.518g); νmax($CH_2Cl_2$) 3520, 3450(br), 1765, 1610, 1525, 1350 $cm^{-1}$.

Example 36

p-Nitrobenzyl 2-[(3RS,4SR)-4-[(1RS)-(E)-3-methoxy carbonyl-1-methyl-prop-2-enyl]-3-[(1SR)-1-(p-nitro benzyloxycarbonyloxy)ethyl]-2-oxo-azetidin-1-yl]-2-(tri phenylphosphoranylidene) acetate.

The product from Example 35 (0.518g) was dissolved in dry tetrahydrofuran (10ml) at -20°C and treated with 2,6-lutidine (0.27ml) followed by thionyl chloride (o.17ml) and the mixture stirred at -20°C for 40 minutes. The solution was filtered and the solvent evaporated. The resulting gum was dissolved in toluene and the solvent evaporated (2 times). The residue, a yellow oil, was dissolved in dioxan (10ml) and treated with 2,6- lutidine (0.27ml) and triphenylphosphine (0.39g). The mixture was stirred at 50°C for 64 hours, filtered, and evaporated under reduced pressure. The residue was chromatographed on Kieselgel 60 (<230 mesh ASTM) using 1:1 ethyl acetate/hexane as eluant to afford the title compound as a pale yellow foam (0.519g); νmax ($CH_2Cl_2$) 1745, 1710, 1650, 1610, 1525, 1350 $cm^{-1}$.

Example 37

(2RS)-2-[(3SR,4SR)-3[(1RS)-1-(p-nitrobenzyloxycarbonyl oxy) ethyl]-1-[(p-nitrobenzyloxycarbonyl)(triphenyl-phosphoranylidene)methyl]-2-oxoazetidin-4-yl]-propanoic acid.

The product from Example 36 was converted into the title compound following the procedure described in Example 13. $\nu$max 1750, 1610, 1590, 1525, 1350cm$^{-1}$.

Example 38

p-Nitrobenzyl 2-[(3RS,4RS)-3-[(1SR)-1-(p-nitrobenzyl oxycarbonyloxy)ethyl-2-oxo-4-[(1SR)-1-(pyrimidin-2-yl thiocarbonyl)-1-ethyl]-azetidin-1-yl]-2-(triphenylphosp horanylidene) acetate.

The product from Example 37 (0.095g) was dissolved in dry tetrahydrofuran (5ml) and treated with triethylamine (0.015g) followed by diethyl chlorophosphate (0.025g) and the reaction mixture stirred at room temperature under an argon atmosphere for 4 hours. The reaction mixture was then treated with lithium pyrimidine-2-thiolate (0.018g) and stirring continued for a further hour. After work-up as described in Example 14, the title compound was obtained as a pale yellow foam (0.073g); νmax($CH_2Cl_2$) 1745, 1610, 1525, 1380, 1350$cm^{-1}$.

0089139

Example 39

p-Nitrobenzyl (4RS, 5SR, 6SR)-4-methyl-6-[(1RS)-1-(p-nitrobenzyloxycarbonloxy)ethyl]-3-(pyrimidin-2-yl thio)-7-oxo-1-azabicyclo [3.2.0]hept-2-ene-2-carboxylate.

The product from Example 38 (0.073g) was dissolved in dry toluene (75ml) and heated to reflux in a Dean and Stark apparatus under an argon atmosphere for 7 hours. The resulting solution was concentrated under reduced pressure and chromatographed on Kieselgel 60 (<230 mesh ASTM) using 8:2 ethylacetate|hexane as eluent. The product was obtained as a colourless oil (0.020g); νmax ($CH_2Cl_2$) 1785, 1750, 1740, 1610, 1565, 1555, 1380, 1350cm$^7$; δ(ppm,$CDCl_3$); 1.15 (3H,d, J=7.4HZ, 4-$CH_3$); 1.49(3H,d,J=6.3HZ, 8-$CH_3$); 3.53(1H,dd,J=2.74, 7.4HZ, 6-H); 4.14-4.26(1H,m,4-H);4.40(1H,dd,J=9.8, 2.74HZ, 5-H); 5.15-5.21(1H,m,8-H); 5.26 (2H,s,benzylic $CH_2$) 5.29 and 5.49 (2H, ABq, J=13.6HZ, benzylic $CH_2$); 7.11(1H,t,J=5.0HZ, pyrimidine); 7.54(2H,d,J=8.8Hz, aromatic);7.64(2H,d,J=8.8HZ, aromatic)8.18-8.24(4H,m, aromatic);8.57 (2H,d,J=5HZ, pyrimidine-H).

Example 40

Sodium (4RS,5SR,6SR)-4-methyl-6-[(1RS)-1-hydroxyethyl]-3-(pyrimidin-2-yl thio)-7-oxo-1-azabicyclo [3.2.0] hept-2-ene-2-carboxylate.

The product from Example 39 (0.020g) was deprotected following the procedure outlined in Example 16, to afford the title compound (0.008g); γ max($H_2O$)297.1nm.

Representative biological data for this compound is as follows.

| ORGANISM | M.I.C(μg/ml) |
|---|---|
| E.coli JT39 | 1.6 |
| S.typhimurium CT10 | 6.2 |
| S.aureus Russell | 0.4 |

CLAIMS:

1. A compound of the formula (II):

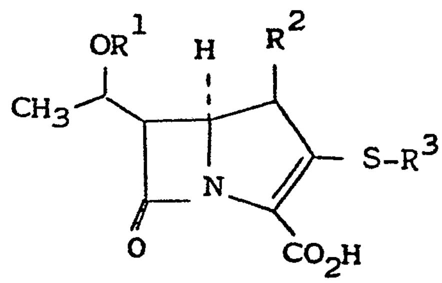

(II)

or a pharmaceutically acceptable salt or pharmaceutically acceptable ester thereof, wherein $R^1$ is hydrogen, -CHO or $-SO_3H$ or a pharmaceutically acceptable salt thereof, $R^2$ is $C_{1-6}$ alkyl or benzyl, and $R^3$ is an optionally substituted pyrimidinyl group.

2. A compound according to claim 1 where $R^2$ is methyl.

3. A compound according to claim 1 or claim 2 wherein $R^3$ is pyrimidin-2-yl.

4. A process for preparing a compound of formula (II)

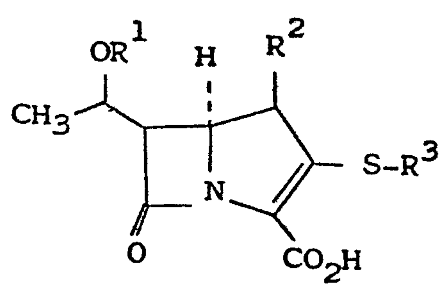

(II)

or a pharmaceutically acceptable salt or pharmaceutically acceptable ester thereof which process comprises the elimination of the elements of $R^4R^5R^6P=O$ from an ester of a compound of formula (IV):

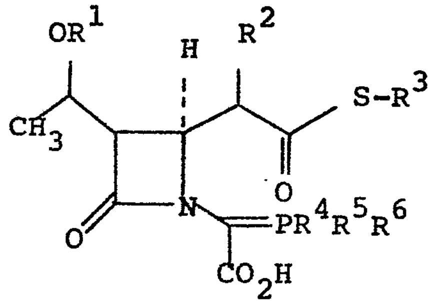

(IV)

wherein $R^1$ is as defined in claim 1 or a protected derivative thereof, $R^2$ and $R^3$ are as defined in claim 1 and $R^4$, $R^5$ and $R^6$ are independently phenyl or methoxyphenyl, and thereafter if necessary:

i) converting a compound of formula (II) wherein $R^1$ is -CHO to a compound of the formula (II) wherein $R^1$ is hydrogen,

ii) converting the ester to an acid, pharmaceutically acceptable salt or pharmaceutically acceptable ester,

iii) removing any protecting group.

5. A pharmaceutical composition comprising a compound of formula (II) as defined in claim 1, or a pharmaceutically acceptable salt or ester thereof and a pharmaceutically acceptable carrier.

6. A synergistic pharmaceutical composition comprising a pharmaceutical composition according to claim 5 and a penicillin or cephalosporin.

7. A method of treating bacterial infection in animals, the method comprising administering a composition according to claim 5 or claim 6.